# EUROPEAN PATENT APPLICATION

(11) **EP 2 787 003 A1**
(43) Date of publication of application: **08.10.2014**
(21) Application number: 13161964.5
(22) Date of filing: 02.04.2013
(51) Int. Cl.: C07K 14/32

(54) **Antimicrobial peptide produced by marine sponge-derived Bacillus subtilis**

(71) Applicant: University College Cork, Cork City (IE)
(72) Inventor: O'Gara, Fergal, Cork (IE); Dobson, Alan, Cork (IE); Barbosa, Teresa, Cork (IE); Phelan, Robert, Cork (IE); Morrissey, John, Cork (IE)
(74) Representative: Purdy, Hugh Barry

(57) **Abstract**

An isolated lantibiotic peptide obtained from marine sponge-derived *B.subtilis* strain MMA7, and having potent antibacterial activity against *Bacillus cereus* and *Listeria monocytogenes,* is described.

## Description

### Field of the Invention

This invention relates to an antimicrobial peptide having potent antibacterial activity against a range of bacteria, a nucleic acid encoding the antimicrobial peptide, a strain of *Bacillus subtilis* bacteria capable of expressing the antimicrobial peptide, an immunity protein conferring resistance to the antimicrobial effects of the antimicrobial peptide and a nucleic acid encoding the cognate immunity protein, and recombinant bacteria transformed with a nucleic acid encoding the antimicrobial peptide and/or a nucleic acid encoding the cognate immunity protein.

### Background to the Invention

The quest for novel antimicrobial compounds from the marine environment has been driven by a widespread resistance to antibiotics and emerging multi-drug resistant pathogens. The large majority of the antimicrobials currently used in clinical therapy are, or are derived from, natural products of microbial origin. While antimicrobials have been traditionally isolated from soil-dwelling microorganisms, the number of bioactive metabolites isolated from marine organisms, and in particular from marine sponges, has increased dramatically in recent years.

Marine sponges (*Porifera*) are simple multicellular, sessile filter feeding invertebrates, which have been in existence for 700-800 million years. They house a dense and diverse microbial population, which is believed to contribute to their unmatched prolific production of bioactive metabolites. These bioactives are thought to play an important protective role against pathogens which might enter the sponge habitat. *Bacillus* species appear to be part of the microbiota of many marine sponges, although their diversity, properties and specific ecological contribution still remain poorly understood (Phelan et al., 2012).

*Bacillus* are rod shaped endospore-forming bacteria renowned for their ability to produce a wide variety of bioactive compounds (Stein, 2005, Westers et al., 2004). The production of antimicrobials coupled with the development of environmentally highly resistant endospores, facilitates their survival in competitive ecological niches such as that which they are likely to encounter within sponges. *B. subtilis* is the best characterised member of the genus, and has for many years been used as a model organism to study cell differentiation in Gram-positive bacteria. Although *B. subtilis* was initially described as a soil dwelling bacterium, numerous reports suggest that this species can be isolated from, and can grow, sporulate and germinate in, many different ecological niches (Barbosa et al., 2004, Barbosa et al., 2005, Phelan et al., 2012). *B*. *subtlis* isolates produce a vast array of chemically different antimicrobial peptides, which include non-ribosomally synthesised peptides, such as polymyxins and the lipopetide surfactin, gene encoded bacteriocins, such as subtilosin and, within this latter group, lantibiotics, such as subtilin. This chemical diversity contrasts with that found for the antimicrobial peptides produced by LAB, which are usually limited to bacteriocins. These antimicrobial peptides are part of the chemical arsenal of many microbes against other microorganisms in nutrient competitive environments or as communicating molecules between different microbial cells.

Lantibiotics are ribosomally synthesised post-translationally modified antimicrobial peptides typically produced by Gram-positive bacteria. Lantipeptides, *i.e*. the name used to describe lantibiotics and related peptides which lack antimicrobial activity, are currently classified into four classes (I to IV) according to the biosynthetic pathway involved in their production. While dedicated dehydratase (LanB) and cyclase enzymes (LanC) are involved in the modification of class I lantibiotics, for the other three classes this is mediated by a single multifunctional enzyme.

The bio-conversion from a linear lantibiotic precursor, with an N-terminal leader peptide and a C-terminal core peptide, to the mature active molecule involves different post-translational modification events of the C-terminal portion, such as the enzymatic conversion of serine and threonine residues into the dehydrated amino acids dehydroalanine (Dha) and dehydroamino-2-butyric acid (Dhb), respectively. When one of these modified amino acids interacts with an intrapeptide cysteine, a thioether bond is formed generating *meso*-lanthionine (Lan) or (2*S*,3*S*,6*R*)-3-methyllanthionine (MeLan) (Cotter et al., 2005a). Besides these, other less common modifications have also been described (Willey and van der Donk, 2007). The post translational modifications appear to influence not only the structure of the peptides, but also their activity and stability against protease degradation and heat denaturation. For class I lantibiotics, the mature peptide is then translocated through the membrane generally by a transmembrane ATP-binding cassette (ABC) transporter (LanT). This can happen before or after cleavage of the leader peptide from the modified core peptide, which is frequently mediated by a dedicated serine protease LanP (Willey and van der Donk, 2007). Multiple roles have been attributed to the leader peptide, which include roles in the post-translational modification events, translocation and immunity. Importantly the peptide only becomes active after removal of the leader peptide. Dedicated immunity proteins (LanI) and/or specialised ABC transporter proteins (LanFE(G)) protect the host from the action of its own lantibiotic. Immunity proteins are thought to be associated with the cell membrane and are heterogeneous in structure, composition and size. All of the genes involved in the processing, transport, immunity and regulation are organised in genetic clusters, which are frequently composed of multiple transcriptional units.

Antimicrobial activity of lantibiotics was initially thought to be limited towards Gram-positive bacteria (Cotter et al., 2005a). However, more recently, as a consequence of the identification of peptides such as microbisporicin, this activity has been extended to Gram-negative bacteria. While the mode of action of most lantibiotics is thought to involve inhibition of peptidoglycan biosynthesis through their interaction with specific molecular targets, such as the cell wall precursor lipid II, many will additionally cause pore formation in the cytoplasmic membrane and ultimately, result in cell death (Willey and van der Donk, 2007). An attractive observation associated with the potential use of lantibiotics as therapeutic drugs is that, to date, the development of microbial resistance to these peptides appears to be relatively less common than is resistance to classical antibiotics (Cotter et al., 2005a).

Lactic acid bacteria (LAB) produce some of the best characterised lantibiotics, including nisin, which remains the only bacteriocin approved for use as a preservative in the food industry (Cotter et al., 2005b). *Bacillus* lantibiotics, despite their broad spectrum of antimicrobial activity, have attracted less attention regarding their potential applicability in food, agricultural and clinical fields. Endospore-forming bacteria, particularly *Bacillus,* are known to play an important role in the preparation of Asian fermented foods and are currently being used as spore probiotic preparations in human therapy, animal production and aquaculture. In this regard, the production of antimicrobial compounds, such as lantibiotics, is regarded as a highly desirable property if candidate strains are to be used as probiotics and/or starter cultures.

### Summary of the Invention

According to the invention, there is provided an isolated peptide, ideally a lantibiotic peptide, having potent antibacterial activity against *Bacillus cereus* and *Listeria monocytogenes* and an N-terminal sequence comprising a peptide of SEQUENCE ID NO: 19 or a functional variant thereof comprising an N-terminal sequence having at least 90% sequence identity with SEQUENCE ID NO: 19.
Dhb*-W-A-Dhb-I-G-K-Dhb-I-V-Q-Dha-V-K-K (SEQUENCE ID NO: 19)
(Dhb* means that the Dhb residue is optionally deaminated)

The invention also relates to an isolated lantibiotic peptide having potent antibacterial activity against *Bacillus cereus* and *Listeria monocytogenes* and which is converted from a linear lantibiotic precursor comprising a peptide of SEQUENCE ID NO: 24 or a variant thereof having at least 90% sequence identity with SEQUENCE ID NO: 24.
T-W-A-T-I-G-K-T-I-V-Q-S-V-K-K (SEQUENCE ID NO: 24)

In the specification, the term "precursor" should be understood to include uncleaved sequences comprising or having a leader peptide and core peptide (for example, SEQUENCE ID NO: 29 or variants thereof) or a sequence in which a core peptide has been cleaved from a leader peptide (for example, SEQUENCE ID NO: 25 or variants thereof). Preferably, the core peptide cleaved from the leader peptide comprises a peptide having an N-terminal sequence of SEQUENCE ID NO: 24 (or variants thereof).

MEKNNIFDLDINKKMESTSEVSAQTWATIGKTIVQSVKKCRTFTCGCSLGSCSN
CN (SEQUENCE ID NO: 29)

The invention also provides an isolated lantibiotic peptide having potent antibacterial activity against *Bacillus cereus* and *Listeria monocytogenes,* the peptide being characterised in that it is obtainable from a marine sponge-derived *B. subtilis* strain, especially marine sponge-derived *B- subtilis* strain MMA7, and has a molecular weight of 3-5KDa as determined by MALDO TOF MS analysis.

The invention also provides an isolated lantibiotic peptide having potent antibacterial activity against *Bacillus cereus* and *Listeria monocytogenes,* the peptide being characterised in that it is obtainable from a marine sponge-derived *B. subtilis* strain, especially as *Haliclona simulans* marine sponge-derived *B- subtilis* strain, and has a molecular weight of 3-5KDa as determined by MALDO TOF MS analysis.

The invention also provides an isolated lantibiotic peptide having potent antibacterial activity against *Bacillus cereus* and *Listeria monocytogenes,* the peptide being characterised in that it is obtainable from a *Haliclona simulans* marine sponge-derived *B- subtilis* strain MMA7, and has a molecular weight of 3-5KDa as determined by MALDO TOF MS analysis.

In one embodiment, the isolated lantibiotic of the invention comprises or consists essentially of, or is converted from, a peptide of SEQ ID NO: 22, or a functional variant thereof having at least 90% sequence identity with SEQ ID NO: 22.
Dhb*-W-A-Dhb-I-G-K-Dhb-I-V-Q-Dha-V-K-K-C-R-Dhb-F-Dhb-C-G-C-Dha-L-G-Dha-C-Dha-N-C-N (SEQ ID NO: 22)
(Dhb* means that the Dhb residue is optionall deaminated)

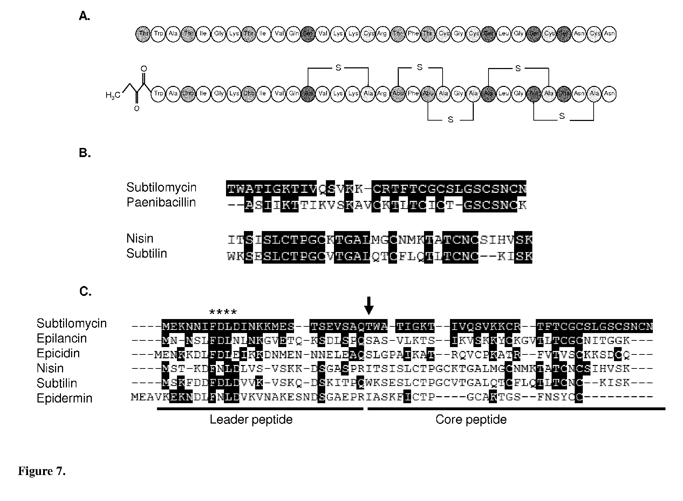

The lantibiotic peptides of the invention, for example those comprising or converted from peptides of SEQUENCE ID NO'S 19 or 22, typically include one or more thioether bridges, for example, two, three, four or five thioether bridges, each of which is independently selected from Lan or MeLan. Thus, the invention also relates to an isolated lantibiotic peptide that is derived from a peptide of the invention, for example a peptide comprising a sequence of SEQUENCE ID NO: 19, 22 or 24, and which includes one or more Lan or MeLan thioeteher bridges. Ideally, the lantibiotic peptide has a sequence and structure shown in Fig. 7A, including five thioether bridges (SEQUENCE ID NO: 43).

Typically, the lantibiotic peptide is further characterised in that it is derived from a linear lantibiotic precursor peptide comprising a peptide of SEQUENCE ID NO: 25 or a variant (or functional variant) thereof having at least 90% sequence identity with SEQUENCE ID NO: 25.
T-W-A-T-I-G-K-T-I-V-Q-S-V-K-K-C-R-T-F-T-C-G-C-S-L-G-S-C-S-N-C-N (SEQUENCE ID NO: 25)

Ideally, the lantibiotic peptide of the invention has an antibacterial activity against *Bacillus cereus* and *Listeria monocytogenes* of at least 40µM, 30µM, 20µM, 10µM, 9µM, 8µM, 7µM, 6µM, 5µM, 4µM, 3µM, 2µM, 1.9µM, 1.8µM, 1.7µM, 1.6µM, 1.5µM, 1.4µM, 1.3µM, 1.2µM, 1.1µM, 1µM, 0.9µM, 0.8µM, 0.7µM, 0.6µM, or 0.5µM.

The invention also relates to a linear lantibiotic precursor of a lantibiotic peptide of the invention. Typically, the linear lantibiotic precursor comprises an N-terminal sequence of SEQUENCE ID NO: 24 or a variant thereof having at least 90% sequence identity with SEQUENCE ID NO: 24. Suitably, the linear lantibiotic precursor comprises SEQUENCE ID NO: 25, or a variant thereof having at least 90% sequence identity with SEQUENCE ID NO: 25.

The invention also relates to a nucleic acid encoding the linear precursor of a lantibiotic peptide of the invention. An example of such a nucleic acid is provided in SEQUENCE ID NO: 23.

The invention also relates to an isolated cognate immunity protein comprising an polyamino acid sequence of SEQ ID NO: 26, or an isolated variant thereof having 90% sequence identity with SEQ ID NO: 26, wherein the isolated protein or variant thereof is capable of conferring immunity on a host strain, typically a *Bacillus* host strain, ideally a *Bacillus subtilis* host strain, to the antibacterial effects of the lantibiotic peptide of the invention.

The invention also relates to a nucleic acid encoding an isolated cognate immunity protein of the invention, or a variant thereof.

The invention also relates to a recombinant vector comprising a nucleic acid of the invention.

The invention also relates to a host cell transformed by a recombinant vector of the invention (hereafter "host cell of the invention").

The invention also relates to an isolated lantibiotic peptide of the invention (or a precursor thereof) for use as a medicament.

The invention also relates to an isolated lantibiotic peptide of the invention (or a precursor thereof) for use as an antibacterial agent or an antibiotic, especially against human and fish-zoonotic pathogens.

The invention also relates to an antimicrobial peptide of the invention (or a precursor thereof) for use in treating or preventing a disease or condition characterised by growth of *Listeria* or *Staphylococcus aureus,* especially MRSA, for example hospital acquired MRSA infection.

The invention also relates to a pharmaceutical composition comprising an isolated lantibiotic peptide of the invention (or a precursor thereof) in combination with a suitable pharmaceutical excipient.

The invention also relates to an antibacterial or antibiotic formulation comprising the isolated lantibiotic peptide of the invention (or a precursor thereof).

The invention also relates to an isolated strain of the invention, or a transformed host cell, of the invention, for use as a probiotic culture.

The invention also relates to an isolated lantibiotic peptide of the invention (or a precursor thereof), for use as a biopreservative, for use in treating microbial infections and clinical infections, for use as a disinfectant, for use as an antibiotic in animal husbandry applications (for example, aquaculture environments), and for use in reducing the incidence of *Aeromonas hydrophila* and sensitive methicillin-resistant S. *aureus* in animals, especially humans and fish.

The invention also relates to a lantibiotic peptide of the invention (or a precursor thereof) in a form suitable for ingestion by aquatic animals, for example fish feed, suitably in a flake or pellet form.

The invention also relates to a method for producing a lantibiotic peptide of the invention comprising a step of culturing a *B. subtilis* strain MMA7 derived from a marine sponge, especially a *Haliclona simulans* marine sponge, under conditions suitable for production of the lantibiotic peptide of the invention, and separating the lantibiotic peptide from the producing strain.

The invention also relates to the use of a *B. subtilis* strain derived from a marine sponge, especially a *Haliclona simulans* marine sponge, typically a *B. subtilis* strain MMA7 derived from a marine sponge, and ideally a *B. subtilis* strain MMA7 derived from a *Haliclona simulans* marine sponge, as an antibacterial agent in the treatment of human or fish-zoonotic pathogens. Typically, the strain is in the form of spores.

### Brief Description of the Drawings

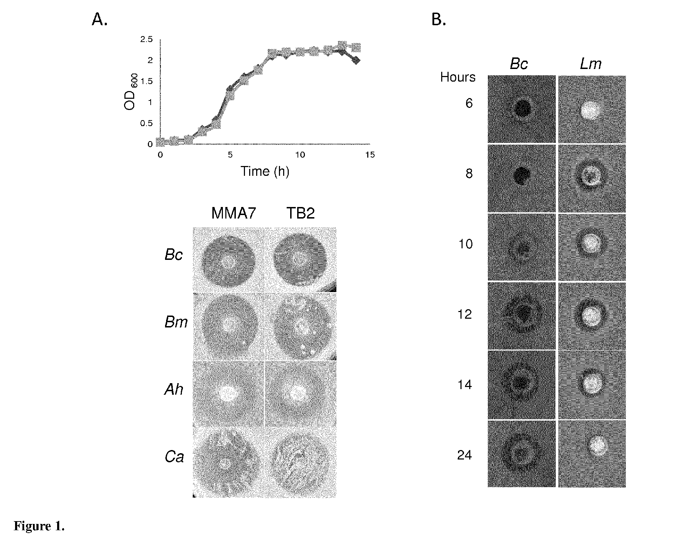

The invention will be more clearly understood from the following description of an embodiment thereof, given by way of example only, with reference to the accompanying drawings, in which:-
Figure 1 illustrates antimicrobial activity of *B. subtilis* strain MMA7. (**A**) Growth of the wild type strain *B. subtilis* MMA7 (diamond) and the *Δsbo-albF::cat* mutant strain TB2 (squares) in MB (top panel). Antimicrobial activity of strains MMA7 and TB2, tested on a colony overlay assay against *B. cereus (Bc), B. megaterium (Bm), A. hydrophila* (*Ah*) and *C*. *albicans (Ca)* (bottom panel). (**B**) Kinetics of production of antimicrobial compounds by *B. subtilis* strain MMA7. Antimicrobial activity of concentrated cell-free supernatants from samples collected at different time points of the bacterial growth was tested on a well diffusion assay against the indicators *B. cereus (Bc)* and *L*. *monocytogenes* (*Lm*). All experiments were repeated twice and representative results are shown.

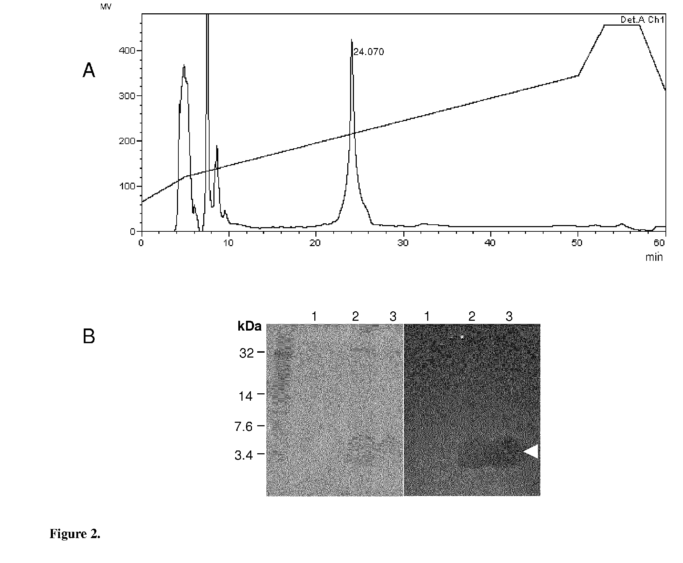

Figure 2 illustrates the purification of the *B. subtilis* strain MMA7 antimicrobial compound. **A**. Reverse phase-HPLC purification of the antimicrobial compound present in ammonium sulphate crude extracts (ASCE) from 12 h MB cultures. A single peak was eluted with an acetonitrile gradient after the injection of 1 ml ASCE (retention time, 24.070 min/46% acetonitrile). **B***.* Bioautography of samples throughout the purification steps. Left panel, Tricine-SDS-PAGE gel stained with Coomassie Blue. Right panel, same as left panel but overlayed with the indicator *C*. *sporogenes.* Lane 1, cell free supernatant; lane 2, ammonium sulphate crude extract; lane 3, RP-HPLC purified bioactive fraction. Arrow head indicates the antimicrobial activity associated with the crude extract and the RP-HPLC purified fraction.

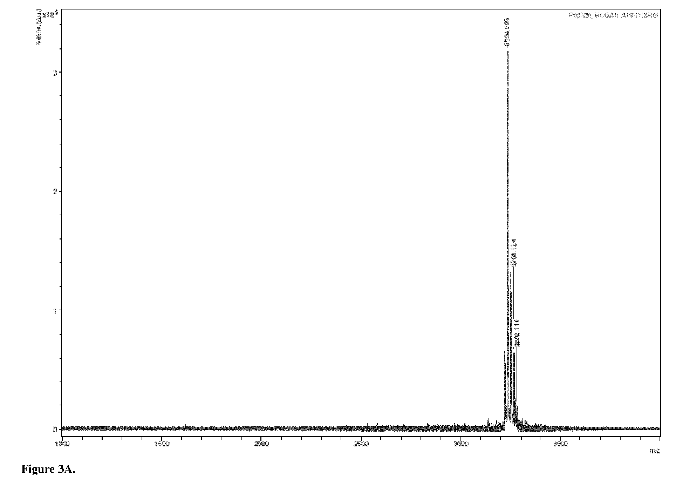

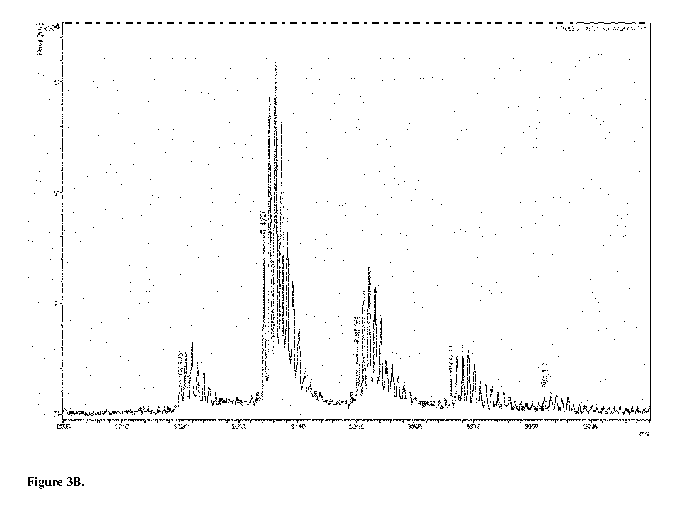

Figure 3 illustrates mass spectrometry analysis of RP-HPLC purified peptide showing a single compound with a low molecular mass, m/z = 3234.2 (**A**). The peptide shows a number of oxidised forms typical of the thio-ethers found in lantibiotics (**B**).

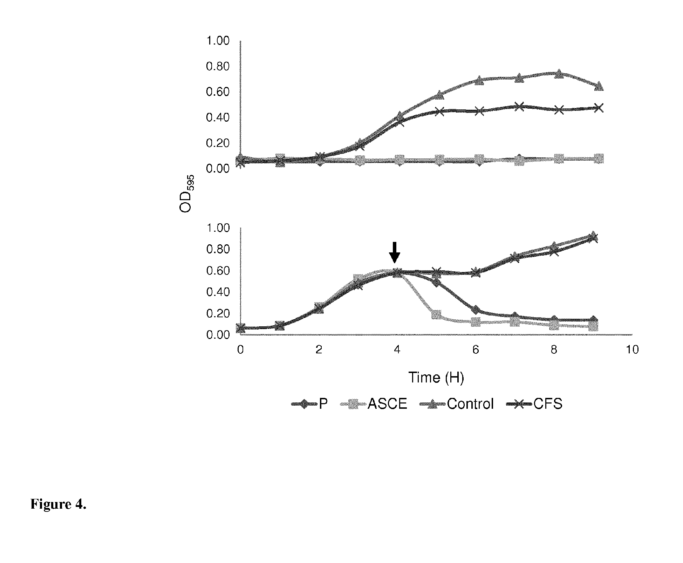

Figure 4 illustrates a bacteriolytic effect of the purified peptide on *L*. *monocytogenes.* Bacterial cultures were grown in the absence (Control) or presence of cell free supernatant (CFS), ammonium sulphate crude extracts (ASCE), and the purified peptide (P), from 12 h MB cultures of strain TB2. Cells were exposed to test samples from the beginning of growth (T0, Top graph) or these were added 4 h after T0 (arrow, Bottom graph).

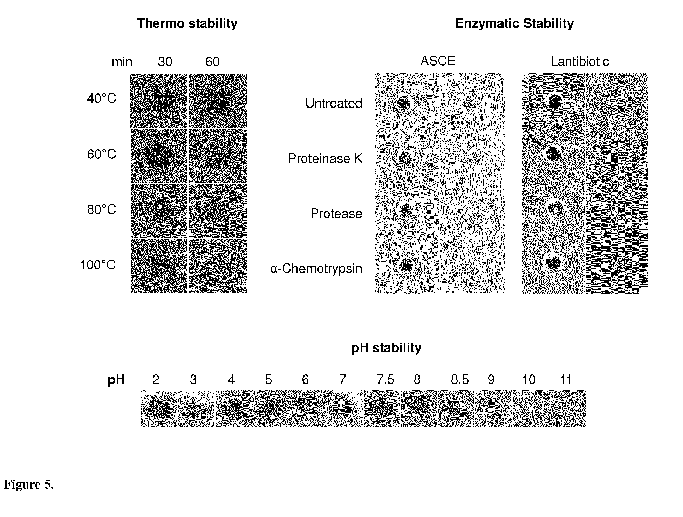

Figure 5 illustrates the thermal, pH and proteolytic stability of the purified peptide. Bioactivity of the bioactive peptide samples treated in the different ways was assessed by a spot on lawn assay. 5 µl of treated/control samples were spotted onto BHI agar plates seeded with *L*. *monocytogenes* to an OD₆₀₀ of 0.015. Samples treated with proteolytic enzymes (1 mg ml⁻¹ final concentration) were tested as above and using a well assay as described above. Control samples containing the different enzymes and no peptide, had no detectable inhibitory effect on the indicator strain. ASCD, ammonium sulphate crude extract. All assays were repeated at least 3 times, and representative results are shown.

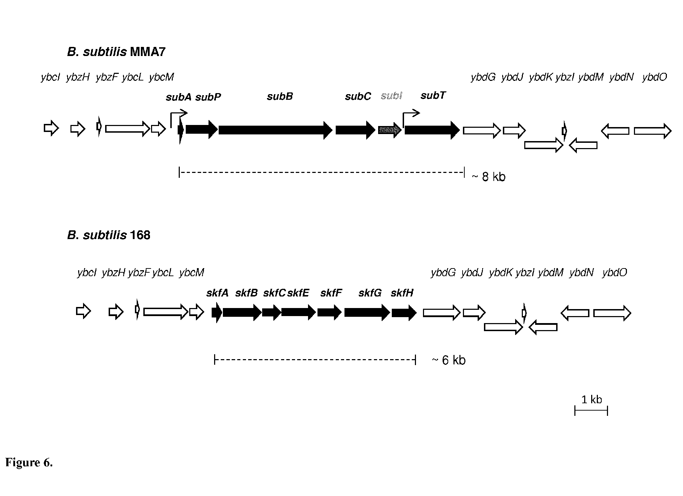

Figure 6 illustrates the structural organisation of the putative subtilomycin biosynthetic cluster and flanking regions. *subA,* subtilomycin structural gene; *sub*P, serine protease; *sub*B, lanthionine dehydratase; *sub*C*,* lanthionine synthetase; *sub*T*,* ABC transporter; *ybdJ,* two component response regulator; *ybdK,* sensor histidine kinase. The function of *sub*I cannot be predicted from its sequence, although its genetic location and lack of homologues is consistent with a possible involvement in immunity. A comparison of the genomic location of the subtilomycin biosynthetic cluster in strain MMA7 with that of the *skf* operon in strain 168 is provided. Predicted promoters are indicated by arrows.
Figure 7 illustrates the comparison of the sequence homology between subtilomycin and nisin pro-petides and their respective closest homologue (**A**). Proposed conformational structure of the mature lantibiotic subtilomycin. Top, unmodified propeptide (SEQUENCE ID NO: 25). Bottom, mature peptide, where Ser and Thr residues which are posttranslationally dehydrated to Dha and Dhb, or involved in the formation of Lan and MeLan, respectively, with cysteine residues, are shaded in grey (SEQUENCE ID NO: 43). The location of the thioether bridges was estimated from the amino acid sequence and by comparison with that of paenibacillin (He et al., 2008). The presence of the N-terminal 2-oxobutyrate residue is also indicated (**B**). Comparison of the amino acid sequence of the N-terminal leader peptide (SEQUENCE ID NO: 28) and the C-terminal core peptide of subtilomycin (SEQUENCE ID NO: 25) (when combined together and uncleaved, comprises the full length protein provided by SEQUENCE ID NO: 29) and different class **I** lantibiotics (**C**). Sequences obtained from http://www.uniprot.org were aligned with ClustalX (Multiple Sequence Alignment version 2.0.11, Larkin et al. 2007) and sequence analysis processed with GeneDoc (Multiple sequence Alignment Editor & Shading Utility, Version 2.7.000, http://www.psc.edu/biomed/genedoc). Conserved amino acids are boxed in black and gaps are indicated by hyphen. +1, indicates the first amino acid of the pro-peptide. *, indicates the conserved motif of class I leader peptides documented to be important for efficient production.

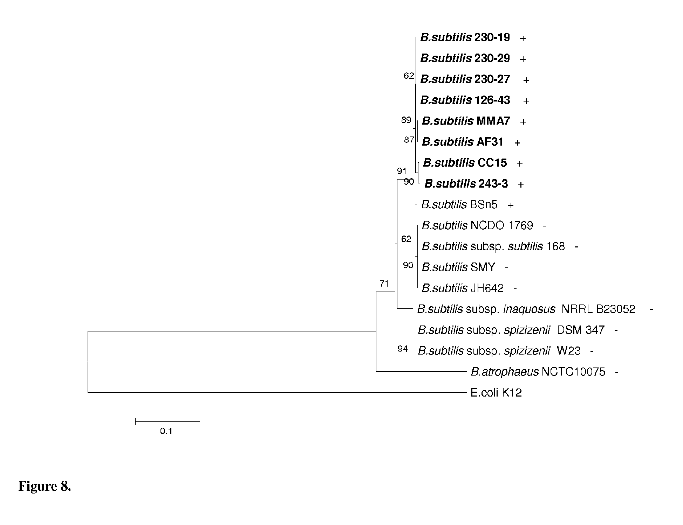

Figure 8 illustrates the neighbour joining phylogenetic tree of *gyrA* gene sequences from different *B. subtilis* strains. Sponge-associated isolates are highlighted in bold. + and -, after strain designation, indicates the presence and absence of the subtilomycin structural gene, *subA.*

### Detailed Description of the Drawings

Broadly, the invention is based on the discovery of an antimicrobial peptide designated Subtilomycin which is a lantibiotic peptide and has potent antibacterial activity against a broad spectrum of bacteria, including the pathogenic *Bacillus cereus* and *Listeria monocytogenes* strains. The amino acid sequence of Subtilomycin in mature/modified form is provided in SEQ ID NO: 23 - the structure of the molecule is shown in Fig. 7A, and include a number of Lan and MeLan modified amino acids. A source of Subtilomycin is a strain of *Baciullus subtilis* that was isolated from the marine sponge *Haliclona simulans,* designated *Bacillus subtilis* strain MMA7 (Phelan et al., 2012). The applicants have also discovered a protein expressed by *Bacillus subtilis* strain MMA7 that confers immunity on its host against the antibacterial effects of Subtilomycin. This protein, designed an "immunity protein" or "cognate immunity protein" has an amino acid sequence provided in SEQ ID NO: 26 and is encoded by the nucleotide sequence provided in SEQ ID NO: 27.

LIQAVLEITIKFLTELISFELPVYMYIWFAVLFIFSMIVENVQLAIAKDSILNSILKR
LGIKILSLLVVLIIGVLGSNFFAQVVEDAVNKESNLAYYILSTTIATALTIIEPIIET
MKKFIKEQSNLAFANFTVSVICLTVTFQAVSSIFSDYFILATPYKVVLIISSAIGIV
YLVKFDQNNLEVNSESVHSNKND (SEQ ID NO: 26)

ttgatacaagcagtactagaaatcactattaaatttctaacggaattaatctcttttgaattgccagtatatatgtatatttggttcgca
gttcttttcatattttcaatgatagtcgaaaatgtacaattggcaatagctaaggatagtatattaaattctattttaaaaagattagga
ataaaaattttaagtctattggtggtcttaattattggggttttaggatcaaactttttttgctcaggttgttgaagacgcagtcaataaa
gaatctaacttagcttattatattctgtctactacaatagctacagcattgaccataatcgaaccaataattgagacgatgaaaaagt
ttataaaagaacaatcaaatcttgcttttgcaaattttacagtcagtgttatttgtcttactgtaacgtttcaagcagtctcaagcatctt
tagtgattattttattttggccaccccttataaagtggtgttaataatttcatcagcaattggtattgtatatttggtgaaattcgatcaaa
ataatttagaagttaatagcgaaagtgttcattcaaataaaaatgattaa (SEQ ID NO: 27)

The term "lantibiotic peptide" as used herein should be understood to mean a polyamino acid sequence, typically of less than 100, 75 or 50 amino acids in length, and which comprises at least one cysteine bridge, typically formed between a dehydrated amino acids selected from dehydroalanine (Dha), dehydroamino-2-butyric acid (Dhb), or α-amino butyric acid (Abu), generating *meso*-lanthionine (Lan) or (2*S*,3*S*,6*R*)-3-methyllanthionine (MeLan). The lantibiotic peptides of the invention comprise at least one, two, three, four, or five thioether bridges, each of which is independently a Lan or MeLan structure. Typically, the lantibiotic peptide of the invention comprises at least two thioether bridges, each of which is independently a Lan or MeLan structure. Suitably, the lantibiotic peptide of the invention comprises at least three thioether bridges, each of which is independently a Lan or MeLan structure. Preferably, the lantibiotic peptide of the invention comprises at least four thioether bridges, each of which is independently a Lan or MeLan structure. Ideally, the lantibiotic peptide of the invention comprises at least two thioether bridges, each of which is independently a Lan or MeLan structure.

The term "variants" as applied herein should be understood to mean peptides having at least 90% sequence identity with the respective reference sequence provided. Suitably, the variants consist of, or comprise, a sequence having at least 91%, 92%, 93%, 94%, 95%, 96%, 97%, 98% or 99% sequence identity with the respective reference sequence. Thus, for example, the term should be taken to include proteins or polypeptides that are altered in respect of one or more amino acid residues, for example 1, 2, 3, 4, 5, 6, 7, 8, 9, or 10 amino acids. Preferably such alterations involve the insertion, addition, deletion and/or substitution of 5 or fewer amino acids, more preferably of 4 or fewer, even more preferably of 3 or fewer, most preferably of 1 or 2 amino acids only. Insertion, addition and substitution with natural and modified amino acids is envisaged. The variant may have conservative amino acid changes, wherein the amino acid being introduced is similar structurally, chemically, or functionally to that being substituted. The term sequence identity comprises both sequence identity and similarity, *i.e.* a polypeptide sequence that shares 90% amino acid identity with SEQ ID NO: 24 is one in which any 90% of aligned residues are either identical to, or conservative substitutions of, the corresponding residues in SEQ ID NO: 24. The term "variant" is also intended to include chemical derivatives of the respective reference peptide, *i.e.* where one or more residues of the respective reference peptide is chemically derivatized by reaction of a functional side group. Also included within the term 'variant' are molecules in which naturally occurring amino acid residues are replaced with amino acid analogues. Details of amino acid analogues will be well known to those skilled in the art. The variant peptide may be a lantibiotic peptide comprising Lan or Melan structures.

The term "functional variant" as applied to as applied herein should be understood to mean variant peptides comprising a sequence having at least 90% sequence identity with a reference sequence and having potent antibacterial activity against *Bacillus cereus* and *Listeria monocytogenes.* In one embodiment, the functional variant is an isolated lantibiotic peptide comprising an N-terminal sequence having at least 90% sequence identity with SEQUENCE ID NO: 19. In another embodiment, the functional variant is an isolated lantibiotic peptide comprising a sequence having at least 90% sequence identity with SEQUENCE ID NO: 22. The peptide will generally have less than 100 residues, 60 residues, 50 residues, 40 residues, 30 residues or 25 residues.. Suitably, the functional variant consist of, or comprise, a sequence having at least 91%, 92%, 93%, 94%, 95%, 96%, 97%, 98% or 99% sequence identity with the reference peptide. Functional variants shall preferably be taken to mean peptides having amino acid sequences which are substantially identical to reference peptides. Thus, for example, the term should be taken to include proteins or polypeptides that are altered in respect of one or more amino acid residues, for example 1, 2, 3, 4, 5, 6, 7, 8, 9, or 10 amino acids. Preferably such alterations involve the insertion, addition, deletion and/or substitution of 5 or fewer amino acids, more preferably of 4 or fewer, even more preferably of 3 or fewer, most preferably of 1 or 2 amino acids only. Insertion, addition and substitution with natural and modified amino acids is envisaged. The variant may have conservative amino acid changes, wherein the amino acid being introduced is similar structurally, chemically, or functionally to that being substituted. Typically, functional variant peptides of the invention, for example functional variants of Subtilomycin peptides of the invention which have been altered by substitution or deletion of residues that are critical to its antibacterial activity will be excluded from the term "variant". The term sequence identity comprises both sequence identity and similarity, *i.e*. a polypeptide sequence that shares 90% amino acid identity with SEQ ID NO: 22 is one in which any 90% of aligned residues are either identical to, or conservative substitutions of, the corresponding residues in SEQ ID NO: 22. The term "variant" is also intended to include chemical derivatives of Subtilomycin peptide, *i.e.* where one or more residues of Subtilomycin is chemically derivatized by reaction of a functional side group. Also included within the term 'variant' are Subtilomycin molecules in which naturally occurring amino acid residues are replaced with amino acid analogues. Details of amino acid analogues will be well known to those skilled in the art. The lantibiotic peptide of the invention comprises thioether bridges, example of which are illustrated in Fig. 7A. It will be appreciated that the lantibiotic peptides of the invention are not limited to the molecule shown in Fig. 7A, but include variants of this molecule having one or more thioether bridges formed between different amino acids in the molecule. In this specification, the term "Dha" should be understood to mean dehydroalanine, the term "Dhb" should be understood to mean dehydroamino-2-butyric acid, the term "Dhb*" should be understood to mean dehydroamino-2-butyric acid in a deaminated form, and the term "Abu" should be understood to mean α-amino butyric acid.

Proteins and polypeptides (including variants and fragments thereof) of and for use in the invention may be generated wholly or partly by chemical synthesis or by expression from nucleic acid. The proteins and peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984)).

The invention also relates to an isolated cognate immunity protein of the invention. This term should be understood to mean a protein of SEQ ID NO: 26, or an isolated variant thereof having 90% sequence identity with SEQ ID NO: 26, wherein the isolated protein or variant thereof is capable of conferring immunity on a host strain, typically a *Bacillus* host strain, ideally a *Bacillus subtilis* host strain, to the antibacterial effects of the lantibiotic peptide of the invention. Thus, a bacteria which expresses an immunity protein of the invention will be immune to the antibacterial effects of the lantibiotic peptide of the invention, or will have increased immunity compared to a bacteria which does not express the immunity protein of the invention. Suitably, the variants comprise a sequence having at least 90%, 91%, 92%, 93%, 94%, 95%, 96%, 97%, 98% or 99% sequence identity with SEQ ID NO: 26.

The invention also relates to a recombinant vector comprising a nucleic acid encoding a lantibiotic peptide of the invention, a nucleic acid encoding a cognate immunity protein of the invention, or one or more nucleic acids encoding a lantibiotic peptide and a cognate immunity protein of the invention. The nucleic acids may be cloned as separate entities (i.e. distinct nucleic acid constructs), or in a same construct, under distinct promoter regions or in a single operon. Typically, the nucleic acids are cloned into a recombinant vector (for example, a plasmid) which is capable of replicating in the host bacteria. Typical plasmids contain, in addition to the cloned insert, a selection gene (*i.e.* antibiotic resistance, a dye *etc*.) and an origin of replication effective in the host bacterium. The plasmid may also comprise regulatory sequences, for example promoters, terminators and/or enhancers. Examples of such vectors are pNZ44 (McGrath S, Fitzgerald GF, van Sinderen D (2001) Improvement and optimization of two engineered phage resistance mechanisms in Lactococcus lactis. Appl. Environ. Microbiol. 67 (2): 608-616)) and pCI372 (Hayes F, Daly C, Fitzgerald GF (1990) Identification of the Minimal Replicon of Lactococcus lactis subsp. lactis UC317 Plasmid pCI305. Appl. Environ. Microbiol. 56: 202-209)). However, any recombinant vector suitable for replicating in a host bacteria known to the person skilled in the art may be used.

The nucleic acid may also be cloned into an integrative cassette suitable for integration into the genome of suitable host bacteria. Such an integrative cassette typically comprises a nucleic acid encoding an antimicrobial peptide of the invention or a cognate immunity protein of the invention, or both, linked to (or flanked by) one or several sequences allowing integration, preferably site-specific integration. Such sequences may be for instance nucleic acid sequences homologous to a targeted region of the genome, allowing integration through crossing over. Various techniques can be used to insert a nucleic acid into a host bacteria, for example through natural transformation or electroporation.

The host bacteria suitable for cloning the lantibiotic peptide and/or the cognate immunity protein may be selected from any host bacteria known to a person skilled in the art such as, for example, *Lactococcus, Lactobacillus* and *Enterococcus.*

In the specification, the term "potent antibacterial activity against *Bacillus cereus* and *Listeria monocytogenes"* as applied to the isolated lantibiotic peptides of the invention should be understood to mean the minimum inhibitory concentration (MIC) required to completely inhibit the growth of *L. monocytogenes* and *C*. *sporogenes.* In this specification, the term "antimicrobial activity of about 1.2 µM against *Listeria monocytogenes* and *Clostridium sporogenes"* as applied to the isolated lantibiotic peptides of the invention should be understood to mean that a concentration of isolated lantibiotic peptides of about 1.2 µM is sufficient to inhibit the growth of bacterial cells following addition of purified peptide to actively growing cells. This concentration is believed to be the minimum inhibitory concentration (MIC) required to completely inhibit the growth of *L. monocytogenes* and *C*. *sporogenes.* Nisin has an MIC of 3.75 µM against *L*. *monocytogenes.* Subtilomycin has an MIC of about 1.2 µM towards *L*. *monocytogenes.* The assay used: A stock sample of purified peptide was diluted in a two-fold serial dilution. 10 µl aliquots of each dilution were applied to agar plates seeded with *L*. *monocytogenes* at an optical density (600 nm) of 0.015. Plates were incubated at 37°C and examined 6 hours post incubation and again at 20 hours post incubation. The minimum inhibitory concentration was determined as the lowest concentration of the peptide that inhibited the visible growth of *L. monocytogenes* after incubation at 37°C after 20 hours.

In one embodiment, the term "cognate immunity protein" should be understood to mean a protein, which, when expressed, increases strain resistance to the lantibiotic peptide when expressed, increases strain resistance to the lantibiotic peptide by increasing the content of esterified D-alanyl groups of techoic acids on the bacterial cell surface thereby resulting in a decrease in the net negative charge of the bacterial cell wall and reducing the potential of the initial electrostatic interaction of the cell with the cationic antimicrobial peptide..

In the specification, the term "isolated" should be considered to mean material removed from its original environment in which it naturally occurs, for example, in this instance a bacterial strain of a marine sponge and/or an antimicrobial peptide designated Subtilomycin and/or a cognate immunity protein. The removed material is typically cultivated, purified and cultured separately from the environment in which it was located. Thus, the purified isolated bacterial strain in this instance ideally does not contain any significant amounts of other bacterial strains. The isolated strain or variant of the invention may be provided in a viable or non-viable form, and in a culturable or non-culturable form, or in the form of spores which may be further treated (for example freeze-dried). The invention also relates to an isolated strain of the invention, or variant thereof, of an antimicrobial peptide designated Subtilomycin and a cognate immunity protein, in any format, for example a freeze-dried form, a suspension, a powder, or a broth, for example a fermentation broth or an extract from a fermentation broth that is enriched in the antimicrobial peptide of the invention.

The term "freeze-dried form" should be understood to mean that the strain, the lantibiotic peptide designated Subtilomycin or the cognate immunity protein of the invention, optionally together with other ingredients including, for example, preservatives, is frozen and then the ice crystals in the frozen strain are sublimated under vacuum.

In the specification, the term "mammal" or "individual" as employed herein should be taken to mean, where appropriate, a human; however it should also include marine animals for which the prophylaxis, therapy or use of the invention is practicable, for example, in fish farms. The term "animal" should be understood to include any animal including humans and marine animals such as, for example, fish.

In this specification, the term "administering" should be taken to include any form of delivery that is capable of delivering the bacterial strain to a site of infection, including local delivery, intravenous delivery, oral delivery, intranasal delivery, intramuscular delivery, intrathecal delivery, transdermal delivery, inhaled delivery and topical delivery. Methods for achieving these means of delivery will be well known to those skilled in the art of drug delivery. For treatment or prophylaxis of MRSA or *Staphylococcus* or *Lysteria* infections, especially chronic MRSA infections in hospital patients, intranasal delivery is ideal as it delivers the bacterial strain expressing the antimicrobial peptide designated Subtilomycin or isolated antimicrobial peptide designated Subtilomycin of the invention directly to the nasal mucous membrane where the target bacteria mucoid infections exist and are ordinarily difficult to remove.

In this specification, the term "pharmaceutical composition" should be taken to mean compositions comprising a therapeutically effective amount of the antimicrobial peptide designated Subtilomycin, or variants thereof, that in one embodiment are produced in-situ in the composition by a bacterial strain, and a pharmaceutically acceptable carrier or diluent. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the bacterial strain and/or antimicrobial peptide is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like.

The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

"Effective amount" refers to the amount or dose of the antimicrobial peptide designated Subtilomycin, or variants thereof, upon single or multiple dose administration to the patient, which provides the desired effect in the patient under treatment. An effective amount can be readily determined by the attending diagnostician, as one skilled in the art, by the use of known techniques and by observing results obtained under analogous circumstances. In determining the effective amount or dose of bacterial strain expressing the antimicrobial peptide or the isolated antimicrobial peptide administered, a number of factors are considered by the attending diagnostician, including, but not limited to: the species of mammal; its size, age, and general health; the specific disease involved; the degree of or involvement or the severity of the disease; the response of the individual patient; the mode of administration; the bioavailabilty characteristics of the preparation administered; the dose regimen selected; the use of concomitant medication; and other relevant circumstances.

The term "comestible product" should be understood to include products that are intended to be consumed by ingestion by humans or animals, such as foods and drinks. In particular, the comestible product is a food or drink product intended for consumption by humans, for example a fermented product or a diary product, especially a fermented dairy product such as a yoghurt.

### Material and Methods

### Bacterial strains, media and growth conditions

Bacterial strains used in this study are listed in Table 1. *B. subtilis* strain MMA7, was isolated from the marine sponge *H. simulans* collected during scuba diving in Gurraig Sound Kilkieran Bay, Galway, on the west coast of Ireland (Phelan *et al.,* 2012). Strains AF31 and CC15 were isolated from the shallow water sponges *Amphilectus fucorum* and *Cliona celata,* respectively, both collected from the Lough Hyne Marine Reserve, Ireland, by Scuba diving. Strains BD230-19, BD230-27, BD230-29, BD243-3 and BD126-43 were isolated from the deep-water sponges BD230, BD126 and BD243, collected from the Rockall Ridge and Porcupine Bank, 300 nautical miles North West of Galway harbour, at a depth of 2900, 2129 and 1300 m, respectively. Unless stated otherwise, marine sponge *B. subtilis* isolates were routinely grown and maintained aerobically on Difco™ Marine agar (MA) and broth (MB) (Difco 2216), at 30°C. Other strains used in this study, including indicator strains, were grown in the media described in Table 1 below, aerobically at 37°C, with the exception of *Aeromonas hydrophila, Vibrio anguillarum, Carnobacterium malteromaticum* and all the *Candida* strains that were incubated at 30°C. *Clostridium perfringens* and *Clostridium sporogenes* were grown anaerobically in an anaerobic jar at 37°C. *E. coli* DH5α used for cloning experiments was grown in Luria-Bertani (LB). For *B. subtilis* strains chloramphenicol was used at 5 µg ml⁻¹.

**Table 1. Bacterial strains used in this study**

| **Bacterial strains** | **Growth media^{a}/origin^{b}** |
|---|---|
| *Bacillus cereus* NCIMB 9373 | LB/ MDCC UCC |
| *Bacillus subtilis* NCDO 1769 | LB/ MDCC UCC |
| *Bacillus subtilis* NRRL B-23052^{T} | LB/ BGSC |
| *Bacillus subtilis* 168 | LB/ BGSC |
| *Bacillus subtilis* DSM 347 | LB/ DSMZ |
| *Bacillus megaterium* ATCC 19213 (BGSC 7A2) | LB/ BGSC |
| *Staphylococcus aureus* NCDO 949 | BHI/ MDCC UCC |
| MRSA ST 530 | BHI/ MDCC UCC |
| VISA 22784 | BHI/ MDCC UCC |
| hVISA 35197 | BHI/ MDCC UCC |
| VRE EC725 | BHI/ MDCC UCC |
| *Euterococcous faecium* NCIMB 11508 | BHI/ MDCC UCC |
| *Listeria innocua* DPC 3567 | BHI/ MDCC UCC |
| *Listeria monocytogenes* EGDe | BHI/ MDCC UCC |
| *Clostridium perfringens* NCDO 1799 | TGB/ MDCC UCC |
| *Clostridium sporogenes* NCDO 1791 | TGB/ MDCC UCC |
| *Clostridium difficile* 001 | BHI/ MDCC UCC |
| *Carnobacterium malteromaticum* LMG 9839 | TSB/ BCCM™ /LMG BC |
| *Lactococcus lactis HP* | GM17/MDCC UCC |
| *Escherichia coli* NCIMB 15943 | LB/ MDCC UCC |
| *Escherichia coli* MUH 103 | LB/ (Mac Aogain et al., 2010) |
| *Enterobacter aerogenes* NCIMB 10102 | LB/ MDCC UCC |
| *Salmonella* Typhimurium LT2 | LB/ MDCC UCC |
| *Pseudomonas aeruginosa* PAO1 | LB/ MDCC UCC |
| *Burkholderia cenocepacia* J2315 | LB/ (Holden et al., 2009) |
| *Stenotrophomonas maltophilia* K279A | LB/ (Crossman et al., 2008) |
| *Klebsiella pneumoniae* MUH 588 | LB/ (Mac Aogain et al., 2010) |
| *Serratia marcescens* MUH 436 | LB/ (Mac Aogain et al., 2010) |
| *Morganella morganii* MUH 988 | LB/ (Mac Aogain et al., 2010) |
| *Aeromonas hydrophila* LMG 2844 | TSB(NA)/ BCCM™ /LMG BC |
| *Listonella* (*Vibrio*) *anguillarum* LMG 4410 | MB/ DSMZ |
| *Candida glabrata* | YPD/ MDCC UCC |
| *Candida albicans* SC5314 | YPD/ MDCC UCC |
| *Candida dubliniensis* | YPD/ MDCC UCC |
| *Candida lusitaniae* | YPD/ MDCC UCC |
| *Candida parapsilosis* | YPD/ MDCC UCC |

^{a}Standard media were: LB, Luria Bertani; TGB, Thioglycolate broth (Merck); YPD, Yeast extract peptone dextrose media; BHI, Brain heart infusion (Merck); MB, Marine broth (Difco); TSB, Tryptic soya broth (Merck); NA, Nutrient agar (Difco). ^{b}Bacterial strains were obtained from the Microbiology Department Culture Collection, University College Cork (MDCC UCC), the *Bacillus* Genetic Stock Centre (BGSC), Belgium Coordinated Collections of Microorganisms (BCCM^{™}) /LGM Bacteria Collection and DSMZ, DSM Collection, Deutsche Sammlung von Mikroorganismen und Zellkulturen GmbH, Braunschweig, Germany. MRSA, *Methicillin resistant Staphylococcus aureus; VISA, vancomycin intermediate Staphylococcus aureus; hVISA,* heterogeneous VISA, *and VRE,* Vancomycin resistant enterococci.

### Antimicrobial activity screening assays

Antimicrobial activity of isolate MMA7 against the indicator strains was initially assessed with a deferred antagonism assay as described by Phelan *et al.* (2012). Subsequent testing of supernatants, crude extracts and purified compounds was carried out with a well and/or a spot on lawn assays. Essentially, 5 µl of samples were spotted onto 20 ml agar plates seeded with the indicator strain to a final OD₆₀₀ of 0.015, or 30 µl aliquots were applied to a 5 mm well made on identical plates.

The concentration of the purified peptide was estimated using the Bicinchoninic acid (BCA) Protein Assay Kit, Pierce^{®}, as described by the manufacturer (Thermo Scientific, Rockford, Illinois, USA).

### DNA extraction, PCR amplification, DNA sequencing and phylogenetic analysis

Genomic DNA was extracted from 5 ml overnight MB cultures by a modification of the guanidine thiocyanate method described by Pitcher et al. (Pitcher et al., 1989). Plasmid DNA was purified from 5 ml overnight *E. coli* LB cultures with the QIAprep spin Miniprep Kit (Qiagen, GmbH, Hilden, Germany).

All oligonucleotide primers used in this study are listed in Table 2 below. Unless stated otherwise PCR reactions (50 µl) contained 1x BioTaq PCR buffer (Bioline), 1.5 mM MgCl₂, 0.2 mM dNTPs, 0.5 µM of each primer, 2.5 U BioTaq DNA polymerase (Bioline), and 3 µl bacterial lysates or 50 ng of genomic DNA as template. With the exception of the annealing temperature (Table 2), and the extension time, the cycling parameters for the amplification of the different target sequences remained constant. An initial denaturation at 94 °C for 4 min, was followed by 30 cycles of 94 °C for 30 s, X °C for 30 s, and 72 °C for X min, with a final extension of 10 min at 72 °C. When required, PCR products were purified using the QIAquick PCR purification kit (Qiagen GmbH, Hilden, Germany). Routine DNA sequencing was performed by GATC-Biotech, AG (Germany).

Partial *gyrA* gene sequences of the marine sponge *B. subtilis* isolates were deposited in GenBank with the following accession numbers: CC15, JX977124; AF31, JX977125; 243-3, JX977126; 230-19, JX977127; 230-29, JX977128; 126-43, JX977129; 230-27, JX977131; MMA7, JX977130. For phylogenetic analysis, 620 nt of the *gyrA* genes (nt 232 to 852 in the *E. coli gyrA* gene, accession number AP012306.1) were aligned using Clustal X2 (Larkin et al., 2007). Neighbour-joining phylogenetic trees were constructed using MEGA 4 (Tamura et al., 2007) and bootstrap tests were performed 1000 times.

**Table 2 Oligonucleotide primers used in this study.**

| **Primer** | **Sequence (5'-3') *** | **Annealing** (°C)/ SEQ ID NO: |
|---|---|---|
| Construction of MMA7 Δ*sbo-albF::cat* mutant | | |
| sboUp-F (HindIII) | CCCAAGCTTCCATCATTGCTCATCAGATTTGA | 58/1 |
| sbo-R (BamHI) | CGCGGATCCTCCGATCGAGCATGTTGCACAA | 58/2 |
| albF-F (EcoRI) | CCGGAATTCCATCCTGCTTGATGCGTTATGGA | 52/3 |
| albF-R (XhoI) | CCGCTCGAGGTGTCTTCTGAGCCTCCGATCAA | 52/4 |

| Verification of MMA7 Δ*sbo-albF::cat* mutant | | |
|---|---|---|
| ywiB-F | CACATGGAGTGTTATCGGTG | 52/5 |
| albFDown-R | CTGTAATCCGGTCCATGTGT | 52/6 |
| albA-R2 | TGCCACAGTTTATGGACGAGAGG | 52/7 |
| albD-F | GCAGACAGAGCAGCAGCTCTGGA | 52/8 |
| cat255-R^{#} | CGTTTGTTGAACTAATGGGTG | 52/9 |
| cat958-D^{#} | GGGTAACTAGCCTGCAGGCAATAGTTACCC | 52/10 |

| Bacteriocins screening primers | | |
|---|---|---|
| spaC-F (subtilin) | ACTATGAATCAATGGAAGG | 48/11 |
| spaS-R (subtilin) | TTGCAGTTACAAGTTAGTG | 48/12 |
| sublancin-F (sublancin) | GTGTGCTGCGTTGCTACAA | 55/13 |
| sublancin-R (sublancin) | TTGACGAGATACAAGCTAGTCC | 55/14 |
| Sbo-F (subtilosin) | GGTTGTGCAACATGCTCGAT | 52/15 |
| AlbA-(subtilosin) | CTCAGGAAGCTGGTGAACTC | 52/16 |
| subA2569-F (subtilomycin) | TGCGGATGACAGATTCGTATTGC | 60/17 |
| subA2571-R (subtilomycin) | ACAGCTGTACCGTGCCCATATAGA | 60/18 |

| | | |
|---|---|---|
| *Introduced restriction sites are underlined. Cat, chloramphenicol resistance cassette. | | |

### Construction of strain MMA7 Δsbo-albF::cat mutant

Preparation and transformation of chemically competent *E. coli* DH5α cells was carried out as described by Sambrook et al.

To create a *sbo-albF* insertion-deletion mutant, DNA fragments containing 284 nt of the upstream and coding region of the structural gene, *sbo* (nt 136 to 420, accession number AJ430547) and 284 nt internal nucleotides of the *albF* gene (nt 5684 to 5968, accession number AJ430547), were PCR amplified from the genomic DNA of strain MMA7 with the primer pair SboUp-F/ Sbo-R and AlbF-F/ AlbF-R, respectively (Table 2). Digested amplified fragments were sequentially inserted between the *Hin*dIII-*Bam*HI sites and *Eco*RI*-Xho*I sites flanking the chloramphenicol resistance cassette in the pMK3 derivative integrational vector, pMS38 (Zilhao et al., 2004). The resulting construct was named pRP1. Strain TB2 (MMA7 Δ*sbo-albF*::*cat*, Chl^{R}), in which the *sbo-albF* region (nucleotides 420 to 5684, accession number AJ430547) was replaced by a chloramphenicol cassette by a double-crossover event, was generated by the transformation of strain MMA7 with *Sca*I linearised pRP1, with subsequent selection for chloramphenicol resistance (Chl^{R}). Disruption of the cluster in the mutants was confirmed by PCR with a combination of primers specific for the chloramphenicol cassette, and primers flanking or internal to, the deleted region. Specifically, primer pairs ywiB-F & albA-R2, ywiB-F & cat255-R, albD-F & albFDown-R, cat958-D & albFDown-R (Table 2).

### Kinetics of subtilomycin production

100 ml MB and LB media were inoculated (1:100) with overnight MB and LB cultures, respectively, of the producing strain and grown at 30°C, 150 rpm shaking on an orbital incubator. The optical density (600 nm) was read hourly until 14h incubation and then at 24h incubation and 5 ml aliquots removed at each time point and centrifuged at 3200 rpm, 15 min, 20°C. Supernatants were filter sterilised (33mm Cellulose Acetate, 0.45 µm syringe filters, Anachem Ltd), and freeze dried (Labconco Freezone6, Labconco Kansas City, *Missouri,* USA) for 16 h. Freeze dried material was resuspended in 500 µl of sterile HPLC grade water (approx. 1/10 initial volume) and tested for activity in a well diffusion assay against different indicator strains.

### Purification and characterisation of subtilomycin

Approximately 1 litre of *B. subtilis* MMA7 12 h MB cultures were centrifuged at 3200 rpm, for 15 min at 20°C and the supernatant filter sterilized using a Nalgene^{®} vacuum filtration system (0.45 µm, Sigma-Aldrich^{®}). Ammonium sulphate was added intermittently to the cell free supernatant to 40 % saturation, and gently stirred overnight at 4°C. The resulting proteinaceous precipitate was collected by centrifugation of 100 ml aliquots at 12,500 rpm, for 90 min, at 4° C and resuspended in total volume of 2 ml of sterile HPLC grade water (Sigma -Aldrich, CHROMASOLV^{®} Plus) (1/500 of original volume). Subsequent purification by Reverse Phase High Performance Liquid Chromatography (RP-HPLC) was achieved by applying 1 ml RP of the ammonium sulphate crude extract onto a Phenomenex^{®} C12 column (Jupiter 4 µ proteo 90 Å, 250 X 10.0, 4 µm). Separation was carried out with a gradient of acetonitrile (33 to 65 %) containing 0.1% TFA from 5 - 50 min, at a flow rate of 2.1 ml min⁻¹ and separation monitored at 214 nm. Fractions were evaporated to dryness on a speedvac (Savant DNA 120, Speedvac, Thermo Scientific), and the resulting pellets resuspended in 1/2 original volume of sterile HPLC grade water, before being tested for activity with a spot on lawn assay against *L. monocytogenes.* The purity and size of the compound on the active fraction was initially estimated on an AXIMA-TOF² MALDI Mass spectrometer (Shimadzu Biotech, Manchester, UK).

Further analysis of the nature and structure of the compound was carried out by Alphalyse A/S (Odense, Denmark). This involved determination of the amino acid composition, MALDI MS and MALDI MS/MS analysis.

1.77 µg of RP-HPLC purified sample resuspended in 0.1% TFA was used for amino acid analysis The acid hydrolysis was performed for 20 hours at 110°C, in 6 N HCl, 0.1% phenol, 0.1% thioglycolic acid. The hydrolysis took place under reduced pressure in an atmosphere of argon. Identification and quantification of the amino acids took place on a BioChrom 30 amino acid analyzer using ion exchange chromatography, post-column derivatization with ninhydrin and detection at two wavelengths, 570 nm and 440 nm. A known amount of the non-natural amino acid norleucine (Nle) was added as an internal control standard. The common 20 amino acids, except tryptophan and cysteine, were determined. Under the conditions employed tryptophan was degraded, and the yield of cysteine was so variable that it was not calculated. Asparagine was determined as aspartic acid and glutamine as glutamic acid.

For MALDI MS and MS/MS of intact peptide, approx. 5 pmol peptide was spotted onto a steel target with alpha-cyano-4-hydroxycinnamic acid (CHCA) matrix and was analyzed on a Bruker Autoflex Speed instrument. For MALDI MS and MS/MS analysis of trypsin digests, the peptide was digested with trypsin at 37 °C overnight and the resulting peptides were concentrated on a C18 ziptip from Millipore and eluted with CHCA matrix in 50% acetonitrile / 0.1% TFA. Analysis was on a Bruker Autoflex Speed instrument. Subsequent analysis involved alkylation of the peptide with β-mercaptoethanol in a basic solution. Alkylation increased the peptide mass by 78 Da for each alkylated amino acid. Samples were taken out at different times to follow the alkylation process. When samples were removed they were acidified with TFA and purified on a C18 ziptip from Millipore before being eluted with alpha-cyano-4-hydroxycinnamic acid matrix in 50% acetonitrile/0.1% TFA. Sample were subsequently analysed by MALDI MS and MS/MS.

Reduction of the peptide with DTT was used to ascertain the potential presence of disulfide bonds, as the peptide mass would be increased by 2 Da when a disulfide bond is broken due to the addition of two hydrogen atoms.

### Tricine-SDS-PAGE Bioactivity Assay

The purity and activity of the bioactive compounds were analyzed by tricine-sodium dodecyl sulfate (SDS)-15% polyacrylamide gel electrophoresis (PAGE) with Tricine running buffer. Samples were run in duplicate, and while one half of the gel was stained with Coomassie blue (Brilliant Blue G, Sigma-Aldrich), the other half was assayed for inhibitory activity by overlaying the gel with soft-agar previously inoculated with a selected indicator strain. Plates were next incubated under the conditions required for the chosen indicator.

### Determination of the mode of action of the purified peptide

Initially 100 µl aliquots of a two-fold serial dilution of the MB 12h cultures CFS, ASCE and the purified peptide (approx. 150 µg ml⁻¹), were applied to individual wells on a 96 flat bottom multiple well plate (Sarstedt). An overnight culture of the indicator strain was diluted to an OD₅₉₅ of 0.05 and 100 µl were applied to each of the wells containing the bioactive peptide. The plate was incubated at 37 °C, with agitation (200 rpm) and the optical density (595 nm) was monitored hourly. Wells where the indicator was added to water only were used as negative controls. Subsequently, 100 µl of indicator overnight cultures diluted to an OD₅₉₅ of 0.05 were applied to individual wells on a 96 well plate and allowed to grow to an OD₅₉₅ of 0.5-0.6. 100 µl of a two-fold serial dilutions of the CFS, the respective ASCE and purified peptide, or water, were then applied to the different wells and growth continued to be monitored hourly. At the different time points samples were taken and the number of cfu determined by plating dilutions onto appropriate agar plates.

### Heat, pH and proteolytic treatment of bioactive samples

To establish the thermal stability of the purified peptide, aliquots were exposed to 40, 60, 80 and 100 °C for 30 and 60 min respectively before being tested for bioactivity on a spot on lawn assay. Untreated samples were used as controls. In a similar way, samples were checked for activity following storage for different time periods at room temperature, 4 °C, -20 °C and -80 °C.

The activity of the purified peptide at different pH values was established by resuspending identical quantities of the peptide in 50 µl of 100 mM Tris buffer, with pH values between 2 to 11. Samples were incubated for 1 h at room temperature before being tested for antimicrobial activity against *L*. *monocytogenes* on spot on lawn assays. The different buffers were also individually tested as negative controls.

Susceptibility to proteolytic enzymes was tested on the ammonium sulphate crude extracts and the purified peptide. Samples resuspended in water were incubated with proteinase K, α-chymotrypsin and a protease mixture from *Streptomyces griseus* (Sigma Aldrich^{®} 81748), to a final concentration of 1 mg ml⁻¹, at 37 °C for 1 h. Samples were then heated to 100 °C for 2 min to inactivate the proteolytic enzymes and bioactivity was tested with a spot on lawn assay.

### Genome sequencing of B. subtilis MMA7 and sequence analysis of the subtilomycin biosynthetic gene cluster

The genome of isolate MMA7 has been sequenced by Genospec, Inc. (Houston, TX USA) using Illumina Sequencing technology on a Illumina High Seq 2000 (Illumina Inc. California, USA). The sequencing data, which provided 100X coverage of the genome, was assembled into 48 contigs, spanning approximately 4.2 Mb. A total of 4277 open reading frames (ORFs) were determined and annotated using the RAST Server (Rapid Annotation using Subsystem Technology) and the Basic Local Alignment

Search Tool (BLAST) programme at NCBI.

BPROM (http://www.softberry.com/berry.phtml) and the Berkeley Drosophila Genome Project (BDGP) (Tomancak et al., 2002, Tomancak et al., 2007), promoter prediction algorithms were used to identify putative promoters within the Subtilomycin gene cluster. Promoters were selected based on the results from both algorithms. Only promoters identified by both algorithms were given further consideration.

For comparison of the amino acid sequence of lantibiotic precursors sequences obtained from htt :11iv.iv.iv..uniprot.org were aligned with ClustalX (Multiple Sequence Alignment version 2.0.11, Larkin et al. 2007) and sequence analysis processed with GeneDoc (Multiple sequence Alignment Editor & Shading Utility, Version 2.7.000, http://www.psc.edu/biomed/genedoc).

### Results

### Spectrum of antimicrobial activity of B. subtilis strain MMA7

Strain *B. subtilis* MMA7 isolated from the marine sponge *H. simulans,* was previously shown to display a strong antimicrobial activity against different indicator bacteria (Phelan et al., 2012). The spectrum of activity of this isolate has been extended in the current study to include marine associated Gram-negative bacteria, such as *Aeromonas hydrophila, Vibrio anguillarum* and *Alteromonas* sp., and nosocomial pathogens, such as VRE, VISA, hVISA and MRSA (Table 1 and Table 4). Strong inhibitory activity was also seen towards multiple *Candida* species, such as *C*. *albicans, C. dubliniensis, C. lusitaniae* and *C. parapsilosis.*

**Table 4. Antimicrobial activity of B. subtilis strain MMA7, derived ammonium sulphate crude extract and purified peptide^{#}.**

| **Indicators** | **Deferred antagonism assay** | **ASCE-12h** | **Peptide** |
|---|---|---|---|
| *B. cereus* | +++ | ++ | ++ |
| *B. megaterium* | ++++ | ++ | ++ |
| *L. monocytogenes* | ++++ | ++ | ++ |
| *L. innocua* | ++++ | ++ | ++ |
| *C. sporogenes* | +++++ | +++ | +++ |
| *C. perfringens* | ++++ | + | - |
| *C. difficile* | ++ | - | - |
| *E. faecium* | ++ | - | - |
| *S. aureus* | ++ | + | + |
| MRSA | ++ | + | + |
| hVISA | + | + | + |
| VRE | + | - | - |
| *L. lactis HP* | ++++ | ++ | + |
| | | | |
| *A. hydrophila* | ++++ | ++ | +* |
| *V. anguillarum* | + | - | +* |
| *Alteromonas sp.* | +++ | + | +* |
| *P. aeruginosa* | - | - | +* |
| | | | |
| *C*. *albicans* | ++++ | - | - |
| *C*. *dubliniensis* | ++ | - | - |
| *C. lusitaniae* | ++ | - | - |
| *C*. *parapsilosis* | + | - | - |

| | | | |
|---|---|---|---|
| ^{#}Producer *B. subtilis* strain MMA7 was grown on Marine agar (MA) for the deferred antagonism assays. Antimicrobial activity of ammonium sulphate crude extracts (ASCE) and purified peptide was tested by a spot on lawn assay. Testing was repeated at least three times and representative results are shown. +, clear halo of growth inhibition; multiple +, increased activity as assessed visually by the increased diameter of the inhibition zone; -, no inhibition. *Activity only detected with 4 to 5 times more peptide than that used for the other indicators. No activity was detected against *Escherichia coli, Enterobacter aerogenes, Salmonella* Typhimurium, *Pseudomonas aeruginosa* PA14, *Burkholderia cenocepacia, Stenotrophomonas maltophilia, Klebsiella pneumoniae, Serratia marcescens, Morganella morganii* and *Candida glabrata.* | | | |

### PCR screening for previously described B. subtilis bacteriocins

In order to test for the presence of genes encoding the *B. subtilis* bacteriocins subtilosin, sublancin or subtilin in strain MMA7, PCR primers sets were designed to amplify sequences corresponding to the associated biosynthetic gene clusters (Table 2). While no amplification was observed with primers targeting sublancin and subtilin, a PCR product was obtained with subtilosin-specific primers. This result was confirmed by sequencing of the 298 bp amplified product [99 °Io nucleotide sequence homology with the subtilosin structural gene, *sbo,* from *B. subtilis* subsp. *subtilis* DSM10 (accession number JN118839) in a blastn search].

To determine if the antimicrobial activity of strain MMA7 was associated with the presumptive production of subtilosin, a deletion insertion mutant was created in which the DNA between the putative peptide structural gene *sbo* and the *albF* peptidase gene, was replaced by a double crossover event by a chloramphenicol resistance cassette, to produce the Δ*sbo-albF::cat* mutant (TB2). The antimicrobial activity of both the wild type strain *B. subtilis* MMA7 and the TB2 mutant was subsequently assessed against susceptible indicator bacteria. No marked difference in either the antimicrobial activity or spectrum of activity was observed between the two *B. subtilis* strains (Fig. 1A), indicating that the putative subtilosin gene in *B. subtilis* MMA7 was not involved in the observed antimicrobial activity and suggesting the likely production of other antimicrobial compound(s) by the strain.

### Kinetics of antimicrobial activity

In order to establish the kinetics of production of the bio-active compound(s), the producing strain was grown in Marine Broth (MB) and Luria Bertani (LB) and the bioactivity of freeze-dried supernatants collected at the different time points examined on a well assay against the indicator *B. cereus* and *L*. *monocytogenes* strains. Inhibitory activity was initially detected at early stationary phase (approx. 8-10h post-incubation) (Figure 1A and 1B). However, this was only the case for MB cultures, with no activity being detected at any time point from LB cultures (data not shown). There was also a clear difference between the spectrum of activity of MB supernatants collected after 12 h incubation versus those collected after 24 h (Figure 1B). While 12 h and 24 h concentrated supernatants showed identical activity against some of the indicators, such as *B. cereus,* the activity detected with 12 h supernatants against *L. monocytogenes,* was not evident from 24 h samples. The latter were also inactive against the indicators C. *sporogenes* and *L*. *lactis.*

### Purification of the antimicrobial compound(s)

The purification strategy for the antimicrobial compound(s) associated with the activity detected from 12 h MB supernatants, involved an initial ammonium sulphate precipitation step (40% saturation). The activity of the resulting precipitate upon resuspension in water suggested that the active compound may be proteinaceous in nature (Table 4, Figure 2B, lane 2). Aliquots (1 ml) of the active ammonium sulphate crude extract (ASCE) were subsequently purified by RP-HPLC on a C12 column, and a single peak with a retention time of 24.070 min, at approximately 46%, on the acetonitrile gradient, was observed (Fig. 2A). The activity of the HPLC fractions containing the peak of interest was next confirmed against a number of indicator strains (Table 4).

In order to establish the purity and size of the bioactive component(s), samples were then run on a 15 % T-SDS-PAGE gel and overlayed with the indicator organism *Clostridium sporogenes.* Activity was associated with a prominent band in the 3 to 5 kDa range, in both the ASCE and the RP-HPLC purified fractions (Figure 2B). A similar activity was observed in *L. monocytogenes* overlays.

MALDI TOF MS analysis of the active RP-HPLC fractions revealed a singular low molecular mass of 3235 Da suggesting that the sample was pure or almost pure (Figure 3A). Searches on different antimicrobial databases returned no match for the proposed mass.

### Antimicrobial spectrum and mechanism of action of the bioactive peptide

The activity of the purified peptide was tested together with its ASCE preparations, against the strains which were initially used as indicators on the colony overlay assay with the producing strain MMA7 (Table 4). While inhibitory activity was readily detected against most of the Gram-positive indicators, the strong activity seen on the colony overlay assay against *V*. *anguillarum, A. hydrophila* and *Alteromonas* sp., although clear, was only detected with four to five fold more peptide than that required to inhibit the Gram-positive indicators. No activity was detected against any of the *Candida* strains.

The nature of the inhibitory activity was further analysed by growing the indicator *L*. *monocytogenes* in the presence or absence of CFS, ASCE and the purified peptide from 12 h MB cultures of the producing strain. While only a small inhibitory effect was observed with the CFS, both the ASCE and subtilomycin preparations appeared to completely abolish growth of the indicator (Figure 4). When test samples were added to *L. monocytogenes* at an OD₅₉₅ of approx. 0.6, a dramatic drop in optical density was detected, which resulted in an equivalent drop in CFUs from aliquots removed at the different time points after the addition of the test samples (Figure 4), suggesting a bacteriolytic action for the peptide. These values were maintained after 24 hr incubation.

### Solubility and stability of the bioactive peptide

Dried pellets originated from 1.5 ml RP-HPLC-purified Subtilomycin containing fractions were fully soluble in water, and aliquots of these samples retained activity against *L*. *monocytogenes* and *B. cereus* on a spot on lawn assay when stored at -20 °C and -80 °C for a period of up to 4 months. No activity was however detected after 5 months storage. Identical samples stored at 4 °C and room temperature lost their activity after two weeks.

The activity of purified Subtilomycin was stable over a wide range of temperatures, including 100 °C for 30 minutes (Figure 5), although no activity against *L*. *monocytogenes* was observed if the samples were incubated for 1 hr at this temperature. Purified subtilomycin was also fully active over a wide pH range (2 to 8), and while a reduction in activity was seen at pH 9, no activity was detected at higher pH values (Figure 5).

Susceptibility to proteolytic degradation was established by mixing 12h ASCE and purified subtilomycin preparations with proteinase K, α-chymotrypsin and a protease from *Streptomyces griseus.* Antimicrobial activity was initially monitored on a spot on lawn assay using *L. monocytogenes* as indicator (Figure 5). None of the treatments resulted in a decrease of activity of the ASCE or the purified subtilomycin, with the exception of a complete loss of activity of the purified subtilomycin after treatment with the *Streptomyces griseus* protease (Figure 5).

### Primary amino acid sequence of subtilomycin

Hydrolysis of the compound purified by RP-HPLC and subsequent amino acid detection confirmed the peptide nature of the compound. Subsequent MS and MS/MS analysis showed that the intact mass of the peptide has a number of oxidised forms (Figure 3B) typical of the presence of thioethers, indicating that the compound was potentially a lantibiotic.

Initial structural analysis of the peptide proved problematic given that Edman degradation was not possible as the N-terminal end of the peptide was blocked. Trypsin digestion and alkylation of the peptide followed by MALDI MS and MS/MS analysis however provided a tentative primary amino acid sequence of the N-terminal part of the peptide; and established that the peptide contained at least 6 to 7 unusual amino acids which are common in lantibiotic peptides, such as Dhb, Dha and lanthionine. By combining data from MS/MS fragmentation of the intact peptide, tryptic peptides from the intact protein and the intact alkylated peptide it was possible to determine up to 15 amino acids from the N-terminal end. Two of the 15 amino acids were Dhb, confirmed by a gain of 78 Da after alkylation and a modified amino acid appeared to be present in the terminal sequence.

The presumptive amino acid sequence provided by the initial structural analysis was used in subsequent BLASTp and TBLASTn searches against the draft genome sequence of *B. subtilis* MMA7. Following theses searches, homology was detected with a small protein-coding gene. This gene is located in the vicinity of protein-coding genes showing similarities to genes encoding lantibiotic modification enzymes. This allowed for the aforementioned experiments to be repeated, refined and the N-terminal sequence of the peptide to be confirmed as T-W-A-T-I-G-K-T-I-V-Q-S-V-K-K (SEQ ID NO: 24). This lantibiotic has been named as 'subtilomycin'. The nucleotide sequence encoding the N-terminal peptide sequence was confirmed as 5'-aaacttgggctacaatcggaaaaactattgtgcagtctgtgaaaaaa-3' (SEQ ID NO: 20).

### Genetic organisation and location of the subtilomycin biosynthetic gene cluster

The primary amino acid sequence of the N-terminal part of subtilomycin obtained from MS/MS *de novo* sequencing was then used to establish the genetic location of the lantibiotic encoding gene. As previously noted, a single match was found with the C-terminal part of a small gene (*subA,* 171 nt), located immediately upstream from ORFs that encode for proteins which have been shown to be involved in the processing of class I lantibiotics, such as LanB-like dehydratase and LanC-like cyclase modification enzymes (SubB and SubC, respectively, Figure 6). These three genes appear to be clustered with three other ORFs, specific to strain MMA7 and to the genome of a recently sequenced endophytic *B. subtilis* strain BSn5, isolated from *Amorphophallus konjac* (Deng et al., 2011), but have relatively low homology to other sequences presented in the NCBI nr database (Table 5). Two of the remaining three ORFs appear to encode a putative extracellular serine protease (*subP*) and an ABC transporter (*subT*) respectively, which are likely to be involved in the processing and transport of the lantibiotic. The other ORF (*subI*) has no homology to any other sequence in the database and could encode an immunity protein. The ORFs flanking these sequences show high homology (greater than 95 %) with the genome of *B. subtilis* 168, including a putative two-component response regulator, *ybdJ,* and sensor histidine kinase, *ybdK,* genes located downstream of the cluster (Figure 6). *In silico* analysis of the biosynthetic cluster identified two putative promoter sequences, located upstream of *subA* and *subT*, respectively.

**Table 5. Sequence homology of the proteins encoded by the subtilomycin biosynthetic gene cluster*.**

| | **No. aa** | **Closest homologue** | **% Identity & aligned region (aa)** | | **E value^{#}** |
|---|---|---|---|---|---|
| SubA | 56 | *Bacillus thuringiensis* serovar *thuringiensis* str. T01001. | 56% | 28/50 | 3E-07 |
| SubP | 324 | *Bacillus amyloliquefaciens* DSM 7 subtilisin-type proteinase | 34% | 113/333 | 6E - 44 |
| SubB | 1045 | *Paenibacillus polymyxa* E681 lantibiotic biosynthesis protein SpaB | 31% | 336/1075 | 6E-144 |
| SubC | 440 | *Bacillus thuringiensis* IBL 200 Lantibiotic biosynthesis protein | 44% | 185/424 | 8E-109 |
| SubI | 80 | *Lactococcus* phage BK5-T hypothetical protein | 31% | 16/51 | 1.3 |
| SubT | 585 | *Staphylococcus epidermidis* VCU121 ABC transporter, ATP-binding protein | 45% | 258/573 | 0.0 |

| | | | | | |
|---|---|---|---|---|---|
| *Results are from a BLASTp search of the GenBank protein database on 12/09/2012. ^{#}Expectation value. | | | | | |

Further sequence analysis of the flanking regions of the putative subtilomycin biosynthetic gene cluster allowed us to establish that the cluster is inserted in the same genomic locus as the sporulation killing factor (*skf*) operon in *B. subtilis* subsp. *subtilis* strain 168 (Gonzalez-Pastor et al., 2003) (Figure 6). The nucleotide sequence encompassing the putative subtilomycin biosynthetic cluster (SEQUENCE ID NO: 21) and neighbouring genes has been deposited in GenBank under accession number JX912247. The amino acid sequence of the putative subtilomycin biosynthetic gene cluster encoded by SEQUENCE ID NO: 21 is provided by SEQUENCE ID NO: 29 (SubA), SEQUENCE ID NO: 30 (SubP), SEQUENCE ID NO: 31 (SubB), SEQUENCE ID NO: 32 (SubC), SEQUENCE ID NO: 33 (SubI), and SEQUENCE ID NO: 34 (SubT).

### Further structural characterisation of subtilomycin

The presence of an N-terminal 2-oxo butyrate residue was established by MS/MS analysis of the peptide. Such residues arise as a consequence of the fact that N-terminal Dhb residues are not stable and undergo spontaneous oxidative deamination. The deaminated Dhb (Dhb*) weighs 85 Da instead of 83 Da, resulting in a mass gain of 2 Da. The mass difference from the alanine at position 3 to the N-terminal of subtilomycin equals 271.3 Da, which corresponds to the sum of 186.07 for the tryptophan residue with 85.03 for the deaminated Dhb.

One sequence for the lantibiotic is Dhb*-W-A-Dhb-I-G-K-Dhb-I-V-Q-Dha-V-K-K-C-R-Dhb-F-Dhb-C-G-C-Dha-L-G-Dha-C-Dha-N-C-N (SEQ ID NO: 22) where the underlined amino acids have been experimentally confirmed by mass spectrometric analysis. The C-terminal of the peptide was not suitable for *de novo* sequencing. This could be due to the presence of the unusual Lan and MeLan amino acids in this region of the peptide. Data from the analysis of tryptic digests suggested that the C-terminal of the peptide could possibly contain up to 5 ring structures, three Lan and two MeLan, since five cysteines, three Dha and two Dhb are present in this section of the peptide. There was no mass change following DTT reductions suggesting that no disulphide bonds were present in the peptide.

In summary, the 32 amino acids long lantibiotic contains nine modified amino acids, five of which are involved in the formation of thioether bridges (Figure 7). The theoretical mass of the proposed sequence is 3234.5, while the observed mass was 3234.6 (m/z = 3235.6).

BLASTp analysis of the peptide against the non-redundant protein sequences (nr) NCBI database did not reveal any homology with previously characterised bacteriocins, retrieving a single 100% homology hit with the previously mentioned endophytic *B*. *subtilis* strain BSn5. The sequence was subsequently used to search the bactibase (http://bactibase.pfba-lab-tun.org/main.php) and the antimicrobial peptide databases (http://aps.unmc.edu/AP/main.php) which highlighted similarities with previously identified lantibiotics. The closest homology (54%) was with paenibacillin produced by *Paenibacillus polymyxa* OSY-DF (SEQ ID NO: 35) (He et al., 2007, He et al., 2008) (Figure 7), followed by epilancin 15X produced by *Staphylococcus epidermidis* 15X154 (43%) (SEQ ID NO: 41). Although the structure of purified paenibacillin has been elucidated, the genetics of the associated biosynthetic cluster have not been published. As a result comparative analysis of the paenibacillin and subtilomycin pre-peptides (Figure 7) and respective gene clusters was not possible.

Based on the primary amino acid sequence of subtilomycin, a proposed structure for subtilomycin is provided (Figure 7), which reflects that experimentally established for paenibacillin (He et al., 2008). However further analysis will be required to definitively establish the proposed structure.

### Distribution of the subtilomycin biosynthetic gene cluster

Different secondary metabolites, and bacteriocins in particular, appear to have distinct distribution or prevalence patterns among related bacteria. A group of *B. subtilis* strains isolated from different coastal (CC15, AF31) and deep water marine (230-19, 230-29, 230-27, 126-43, 243-3) sponges were thus screened with PCR primers targeting the subtilomycin structural gene, *subA* (Table 2). A PCR product was detected in all of the marine sponges tested, but was absent in other non-marine derived *B. subtilis* strains which were screened (Figure 8). In order to establish the phylogenetic relationship between the sponge isolates with their counterparts, the *gyrA* gene sequences obtained from the strains, using *gyrA* specific PCR primers (Table 2) were used to generate a Neighbour-joining phylogenetic tree (Figure 8). This analysis indicated that the sponge isolates although, more closely related to *B. subtilis* subsp. *subtilis* than to the other subspecies, appear to form subgroups which cluster together, and are more closely related to each other than to other, non-sponge, isolates.

Despite several reports describing the isolation of bioactive *Bacillus* strains from marine sponges, very few have successfully identified the associated antimicrobial compounds. In this study a novel class I lantibiotic, herein named subtilomycin, produced by *B*. *subtilis* MMA7 isolated from the marine sponge *Haliclona simulans* has been isolated and characterised.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

### References

Barbosa, T. M., C. R. Serra, and A. O. Henriques. 2004. Gut Sporeformers. Pages 183-191 in Bacterial spore formers: probiotics and emerging applications. E. Ricca, A. O. Henriques, and S. M. Cutting, ed. Horizon Bioscience, Norfolk, United Kingdom. Barbosa, T. M., C. R. Serra, R. M. La Ragione, M. J. Woodward, and A. O. Henriques. 2005. Screening for Bacillus isolates in the broiler gastrointestinal tract. Appl Environ Microbiol 71(2):968-978.
Cotter, P. D., C. Hill, and R. P. Ross. 2005a. Bacterial lantibiotics: strategies to improve therapeutic potential. Curr Protein Pept Sci 6(1):61-75.
Cotter, P. D., C. Hill, and R. P. Ross. 2005b. Bacteriocins: developing innate immunity for food. Nat Rev Microbiol 3(10):777-788.
Crossman, L. C., V. C. Gould, J. M. Dow, G. S. Vernikos, A. Okazaki, M. Sebaihia, D. Saunders, C. Arrowsmith, T. Carver, N. Peters, E. Adlem, A. Kerhornou, A. Lord, L. Murphy, K. Seeger, R. Squares, S. Rutter, M. A. Quail, M. A. Rajandream, D. Harris, C. Churcher, S. D. Bentley, J. Parkhill, N. R. Thomson, and M. B. Avison. 2008. The complete genome, comparative and functional analysis of Stenotrophomonas maltophilia reveals an organism heavily shielded by drug resistance determinants. Genome biology 9(4):R74.
Gonzalez-Pastor, J. E., E. C. Hobbs, and R. Losick. 2003. Cannibalism by sporulating bacteria. Science (New York, N.Y.) 301(5632):510-513.
He, Z., D. Kisla, L. Zhang, C. Yuan, K. B. Green-Church, and A. E. Yousef. 2007. Isolation and identification of a Paenibacillus polymyxa strain that coproduces a novel lantibiotic and polymyxin. Appl Environ Microbiol 73(1):168-178.
He, Z., C. Yuan, L. Zhang, and A. E. Yousef. 2008. N-terminal acetylation in paenibacillin, a novel lantibiotic. FEBS letters 582(18):2787-2792.
Holden, M. T., H. M. Seth-Smith, L. C. Crossman, M. Sebaihia, S. D. Bentley, A. M. Cerdeno-Tarraga, N. R. Thomson, N. Bason, M. A. Quail, S. Sharp, I. Cherevach, C. Churcher, I. Goodhead, H. Hauser, N. Holroyd, K. Mungall, P. Scott, D. Walker, B. White, H. Rose, P. Iversen, D. Mil-Homens, E. P. Rocha, A. M. Fialho, A. Baldwin, C. Dowson, B. G. Barrell, J. R. Govan, P. Vandamme, C. A. Hart, E. Mahenthiralingam, and J. Parkhill. 2009. The genome of Burkholderia cenocepacia J2315, an epidemic pathogen of cystic fibrosis patients. J Bacteriol 191(1):261-277.
Larkin, M. A., G. Blackshields, N. P. Brown, R. Chenna, P. A. McGettigan, H. McWilliam, F. Valentin, I. M. Wallace, A. Wilm, R. Lopez, J. D. Thompson, T. J. Gibson, and D. G. Higgins. 2007. Clustal W and Clustal X version 2.0. Bioinformatics (Oxford, England) 23(21):2947-2948.
Mac Aogain, M., M. J. Mooij, C. Adams, J. Clair, and F. O'Gara. 2010. Emergence of extended-spectrum beta-lactamase and fluoroquinolone resistance genes among Irish multidrug-resistant isolates. Diagnostic microbiology and infectious disease 67(1):106-109.
Phelan, R. W., O. H. JA, J. Kennedy, J. P. Morrissey, A. D. Dobson, F. O'Gara, and T. M. Barbosa. 2012. Diversity and bioactive potential of endospore-forming bacteria cultured from the marine sponge Haliclona simulans. J Appl Microbiol 112:65-78. Pitcher, D. G., N. A. Saunders, and R. J. Owen. 1989. Rapid extraction of bacterial genomic DNA with guanidium thiocyanate. Lett. Appl. Microbiol 8:151-156.
Sahl, H. G., R. W. Jack, and G. Bierbaum. 1995. Biosynthesis and biological activities of lantibiotics with unique post-translational modifications. Eur J Biochem 230(3):827-853.
Stein, T. 2005. Bacillus subtilis antibiotics: structures, syntheses and specific functions. Mol Microbiol 56(4):845-857.
Tamura, K., J. Dudley, M. Nei, and S. Kumar. 2007. MEGA4: Molecular Evolutionary Genetics Analysis (MEGA) software version 4.0. Mol Biol Evol 24(8):1596-1599. Taylor, M. W., R. Radax, D. Steger, and M. Wagner. 2007. Sponge-Associated Microorganisms: Evolution, Ecology, and Biotechnological Potential. Microbiol. Mol. Biol. Rev. 71(2):295-347.
Tomancak, P., A. Beaton, R. Weiszmann, E. Kwan, S. Shu, S. E. Lewis, S. Richards, M. Ashburner, V. Hartenstein, S. E. Celniker, and G. M. Rubin. 2002. Systematic determination of patterns of gene expression during Drosophila embryogenesis. Genome biology 3(12):RESEARCH0088.
Tomancak, P., B. P. Berman, A. Beaton, R. Weiszmann, E. Kwan, V. Hartenstein, S. E. Celniker, and G. M. Rubin. 2007. Global analysis of patterns of gene expression during Drosophila embryogenesis. Genome biology 8(7):R145.
Westers, L., H. Westers, and W. J. Quax. 2004. Bacillus subtilis as cell factory for pharmaceutical proteins: a biotechnological approach to optimize the host organism. Biochim Biophys Acta 1694(1-3):299-310.
Willey, J. M. and W. A. van der Donk. 2007. Lantibiotics: peptides of diverse structure and function. Annu Rev Microbiol 61:477-501.
Zilhao, R., M. Serrano, R. Isticato, E. Ricca, C. P. Moran, Jr., and A. O. Henriques. 2004. Interactions among CotB, CotG, and CotH during assembly of the Bacillus subtilis spore coat. J Bacteriol 186(4):1110-1119.

## Claims

1. An isolated lantibiotic peptide having potent antibacterial activity against *Bacillus cereus* and *Listeria monocytogenes* and an N-terminal sequence comprising a peptide of SEQUENCE ID NO: 19 or a functional variant thereof comprising an N-terminal sequence having at least 90% sequence identity with SEQUENCE ID NO: 19.

2. An isolated lantibiotic peptide of Claim 1 further **characterised in that** it is derived from a linear lantibiotic precursor comprising a peptide of SEQUENCE ID NO: 24 or a variant thereof having at least 90% sequence identity with SEQUENCE ID NO: 24.

3. An isolated lantibiotic peptide of Claim 1 or 2 and comprising a peptide of SEQ ID NO: 22, or a functional variant thereof having at least 90% sequence identity with SEQ ID NO: 22.

4. An isolated lantibiotic peptide of Claim 1, 2 or 3 further **characterised in that** it is derived from a linear lantibiotic precursor peptide comprising a peptide of SEQUENCE ID NO: 25 or a variant thereof having at least 90% sequence identity with SEQUENCE ID NO: 25.

5. An isolated lantibiotic peptide of any preceding Claim and having five thioether bridges.

6. An isolated linear lantibiotic peptide precursor of the lantibiotic peptide of any of Claims 1 to 5.

7. A nucleic acid encoding an isolated linear lantibiotic peptide precursor of Claim 6.

8. A recombinant vector comprising a nucleic acid of Claim 7.

9. A host cell transformed by a recombinant vector of Claim 8.

10. An isolated lantibiotic peptide of any of Claims 1 to 5, or isolated linear lantibiotic precursor of Claim 6, for use as an antibacterial agent or an antibiotic against human and fish-zoonotic pathogens.

11. An antimicrobial peptide of any of Claims 1 to 5, or isolated linear lantibiotic precursor of Claim 6, for use in treating or preventing a disease or condition **characterised by** growth of hospital acquired MRSA infection.

12. A fish feed comprising a lantibiotic peptide of any of Claims 1 to 5 or isolated linear lantibiotic precursor of Claim 6.

13. A method for producing a lantibiotic peptide of any of Claims 1 to 5 comprising a step of culturing a *B. subtilis* strain MMA7 derived from a marine sponge under conditions suitable for production of the lantibiotic peptide and separating the lantibiotic peptide from the producing strain, in which the *B.subtilis* strain MMA7 is optionally derived from a *Haliclona simulans* marine sponge.

14. A *B. subtilis* strain MMA7 derived from a marine sponge, for use as an antibacterial agent in the treatment of human or fish-zoonotic pathogens, in which the *B.subtilis* strain MMA7 is optionally derived from a *Haliclona simulans* marine sponge.

15. An isolated cognate immunity protein comprising an polyamino acid sequence of SEQ ID NO: 26, or an isolated variant thereof having 90% sequence identity with SEQ ID NO: 26, wherein the isolated protein or variant thereof is capable of conferring immunity on a *Bacillus subtilis* host strain, to the antibacterial effects of a lantibiotic peptide of Claim 1.


SEQUENCE LISTING

<110> UNIVERSITY COLLEGE CORK - NATIONAL UNIVERSITY OF IRELAND,
      CORK

<120> ANTIMICROBIAL PEPTIDE PRODUCED BY MARINE SPONGE-DERIVED BACILLUS
      SUBTILIS

<130> P11096EP00

<140>
<141>

<160> 43

<170> PatentIn version 3.5

<210> 1
<211> 32
<212> DNA
<213> Artificial Sequence

<220>
<221> source
<223> /note="Description of Artificial Sequence: Synthetic
      oligonucleotide"

<400> 1
cccaagcttc catcattgct catcagattt ga                                    32


<210> 2
<211> 31
<212> DNA
<213> Artificial Sequence

<220>
<221> source
<223> /note="Description of Artificial Sequence: Synthetic
      oligonucleotide"

<400> 2
cgcggatcct ccgatcgagc atgttgcaca a                                     31


<210> 3
<211> 32
<212> DNA
<213> Artificial Sequence

<220>
<221> source
<223> /note="Description of Artificial Sequence: Synthetic
      oligonucleotide"

<400> 3
ccggaattcc atcctgcttg atgcgttatg ga                                    32


<210> 4
<211> 32
<212> DNA
<213> Artificial Sequence

<220>
<221> source
<223> /note="Description of Artificial Sequence: Synthetic
      oligonucleotide"

<400> 4
ccgctcgagg tgtcttctga gcctccgatc aa                                    32


<210> 5
<211> 20
<212> DNA
<213> Artificial Sequence

<220>
<221> source
<223> /note="Description of Artificial Sequence: Synthetic
      oligonucleotide"

<400> 5
cacatggagt gttatcggtg                                                  20


<210> 6
<211> 20
<212> DNA
<213> Artificial Sequence

<220>
<221> source
<223> /note="Description of Artificial Sequence: Synthetic
      oligonucleotide"

<400> 6
ctgtaatccg gtccatgtgt                                                  20


<210> 7
<211> 23
<212> DNA
<213> Artificial Sequence

<220>
<221> source
<223> /note="Description of Artificial Sequence: Synthetic
      oligonucleotide"

<400> 7
tgccacagtt tatggacgag agg                                              23


<210> 8
<211> 23
<212> DNA
<213> Artificial Sequence

<220>
<221> source
<223> /note="Description of Artificial Sequence: Synthetic
      oligonucleotide"

<400> 8

gcagacagag cagcagctct gga                                              23

<210> 9
<211> 21
<212> DNA
<213> Artificial Sequence

<220>
<221> source
<223> /note="Description of Artificial Sequence: Synthetic
      oligonucleotide"

<400> 9
cgtttgttga actaatgggt g                                                21

<210> 10
<211> 30
<212> DNA
<213> Artificial Sequence

<220>
<221> source
<223> /note="Description of Artificial Sequence: Synthetic
      oligonucleotide"

<400> 10
gggtaactag cctgcaggca atagttaccc                                       30

<210> 11
<211> 19
<212> DNA
<213> Artificial Sequence

<220>
<221> source
<223> /note="Description of Artificial Sequence: Synthetic
      oligonucleotide"

<400> 11
actatgaatc aatggaagg                                                   19

<210> 12
<211> 19
<212> DNA
<213> Artificial Sequence

<220>
<221> source
<223> /note="Description of Artificial Sequence: Synthetic
      oligonucleotide"

<400> 12
ttgcagttac aagttagtg                                                   19

<210> 13
<211> 19
<212> DNA
<213> Artificial Sequence

<220>
<221> source
<223> /note="Description of Artificial Sequence: Synthetic
      oligonucleotide"

<400> 13
gtgtgctgcg ttgctacaa                                                    19


<210> 14
<211> 22
<212> DNA
<213> Artificial Sequence

<220>
<221> source
<223> /note="Description of Artificial Sequence: Synthetic
      oligonucleotide"

<400> 14
ttgacgagat acaagctagt cc                                                22


<210> 15
<211> 20
<212> DNA
<213> Artificial Sequence

<220>
<221> source
<223> /note="Description of Artificial Sequence: Synthetic
      oligonucleotide"

<400> 15
ggttgtgcaa catgctcgat                                                   20


<210> 16
<211> 20
<212> DNA
<213> Artificial Sequence

<220>
<221> source
<223> /note="Description of Artificial Sequence: Synthetic
      oligonucleotide"

<400> 16
ctcaggaagc tggtgaactc                                                   20


<210> 17
<211> 23
<212> DNA
<213> Artificial Sequence

<220>
<221> source
<223> /note="Description of Artificial Sequence: Synthetic
      oligonucleotide"

<400> 17

tgcggatgac agattcgtat tgc                                                23

<210> 18
<211> 24
<212> DNA
<213> Artificial Sequence

<220>
<221> source
<223> /note="Description of Artificial Sequence: Synthetic
      oligonucleotide"

<400> 18
acagctgtac cgtgcccata taga                                               24

<210> 19
<211> 15
<212> PRT
<213> Bacillus subtilis

<220>
<221> MOD_RES
<222> (1)..(1)
<223> Dhb

<220>
<221> MOD_RES
<222> (4)..(4)
<223> Dhb

<220>
<221> MOD_RES
<222> (8)..(8)
<223> Dhb

<220>
<221> MOD_RES
<222> (12)..(12)
<223> Dha

<400> 19
Xaa Trp Ala Xaa Ile Gly Lys Xaa Ile Val Gln Xaa Val Lys Lys
1               5                   10                  15

<210> 20
<211> 47
<212> DNA
<213> Bacillus subtilis

<400> 20
aaacttgggc tacaatcgga aaaactattg tgcagtctgt gaaaaaa                      47

<210> 21
<211> 11100
<212> DNA
<213> Bacillus subtilis

<400> 21

tggtcgtttg ccgcatggtc caccggtccg acacagcaat ttcacctggc tacaatagaa 60

ccggaaatgt caggtgtttt gcttagcatg aatcagtcaa tgatgcaatt cgccatggcg 120

gtcggcgcag gtataggagg ggtttttgtg gaaaacgtat cattggcctc gattacctgg 180

gttggtgcgt taggggttat gattgccatt attgcatcat tgctgatttt caattcgcaa 240

ccgaaacaag cgctaaaaga tatcaatcaa taatttcgaa agttctaaca tccgctcgtt 300

atacaagcgg gtgttttttt agcgttcaaa gtcagtccat gcatgagccc tccaagggcc 360

gacatcgttc tgtatgtgtc gaaggcgcac tgaagctgaa tgagatttct taaggctttg 420

ccgcggcgag ctgaagtaca gtacgattgc cttgatcgaa cactggaaac acaagagaac 480

gtaaacctga acatccgcgt aaacgtcaag gaagttgcca cttggggagt aaacacttgc 540

atcatctcgc tgaaaggcct agacaatgcg gatgacagat tcgtattgcc ggaagtaaac 600

acagcgcttg ctctgtttcc gttgtcgatt gcagctgatt gcttactaat gctgccctgc 660

atctcggttg tgatgtcgat aagcagcgta accttgtgaa gagtgttact gtggagtaag 720

atttttaacc cctttggaat atagatcaaa aggtttttta atacttataa actgaaggag 780

atcaatttta aatcgtttta aaactattaa aggaagttta gtaacgataa cttaaacatc 840

tggctaataa gattactgag gatacatcaa aagtcgaata gtaatagtag agagtaaaag 900

taaccgtaag aagaattata acaattagtt agcaaataat taaaaaatgt cccaaaaaaa 960

gtattgacta actttttttg ggatagtact attgaattgc aaccggttga aagtatcaaa 1020

aaaaggagga aatggaaatg gagaagaata atatttttga cctagacatt aataaaaaga 1080

tggaatctac tagtgaggtt tctgctcaaa cttgggctac aatcggaaaa actattgtgc 1140

agtctgtgaa aaaatgtaga acatttactt gtggatgttc gctaggctct tgcagtaact 1200

gtaactaatt aggggatgag cagagggagt taaaatacat actatattaa aactccctct 1260

tcttctaaag acaaggagta aacaattcca taaatatgaa tatgactaca aatgctttac 1320

ctaaccaatt ttcttggaac tcttataaag taacaaatgg atataggcta tatgatcttc 1380

aagttgggag ccctttaatt aagatagctg taattgatag tgggtatgat cctaatcatt 1440

ccgagattac tggaaatatt gataaaaaaa aatcttgttc attcgttgat tatgagaatt 1500

ttaatgaaga tctatatggg cacggtacag ctgttattag tgtgattgta gggaaaaatc 1560

aagttaaagg agtagctccc ggaattacaa ttattagtta taaagttcta gatagaaatg 1620

gttttgggaa aatagaatgg gtaatacatg ctattttaaa agccattgaa gattgtgtcc 1680

acattataaa tttaagtcat gtttttacta tgttcggatt gggaaataaa ggagcattca 1740

ataaacttca gcaggccatt gataaagcca gaaagaaagg cataataatt gtaggggcag 1800

cagggaatca aggagtttta ttagatatga agagtacaag tttataccct acaattaagg 1860

gaattaacta tgtcacctca acaaataaaa ataattacac ctcatcttac gcaaatattg 1920

ggtcaattat tcgatttgct gcaccatcag gggaacttga tgaaacaaat aatgttacag 1980

acttaataat agttgcacat cccacaaatt ttccaaaaac aaagtttgaa aaagcaattg 2040

ggcttccgga cttttataca gttacatgtg gaactagtat atcagcagct atgttttcga 2100

gtctaatagc attagtattg actgaatatt tgatatgtaa gcaaattcac ctatcagaaa 2160

aagaaattct taatataatc caaaaaaact cctcacgagt aaaagatcaa aattctattt 2220

tcgaaataaa tgcttatagt gtaataaaat ttatcaaagg attgttataa atgaatacaa 2280

aatatttaag ctcagacttg tttatgtata gaaagccgat attggactta tcgttttata 2340

aagagtgttt agcttttgat aatgaattta acgaagagga atacatagcg aagataagta 2400

acttattaaa cactaatccc actatagcag aggccattta ttttgcaagt gaaccactat 2460

ataattcttt tttaaatata gaaaaattaa aaaagaaaaa gcgcaagagt gttattgata 2520

gtatgctaaa gtttttaata agaatgagta caagaccaac tccatttggc cttttttcag 2580

gtattggggt tgaaacattt tcaggtcagg atgaatattt aactaaagat tctgaagtta 2640

taaaaaatac tcgtttggat atgtgttgga tctatttctt aataagaaaa ttagagagta 2700

accttagtat tctcgagcag ttaactgtgg aaaataactc actggtttat aatcacggtg 2760

atagaatcaa attaagttat caaagtctat tacacagtga cgataatgat cagtcattag 2820

aaatgttaac ccttagaaag acagcagtaa tggattatgt ctattcgcaa gcagaattcg 2880

gtatcaatta ctctacgtta agagatagta taaataaaac atatagtgtt actgatgaca 2940

tatcaaatga ttttttaaat agtctattaa aaaatgaagt tattataact aacttaagaa 3000

catctcttgt taattctgat ccttttgaaa atatattaaa cataattagt aaaatgcata 3060

atgtagagga agaattgaat tatttagttc gattaaaaaa atcgattgaa gaatacaaaa 3120

aatcttctgg ggaattaggt ataagtattt taaagaaaat caaaaaggaa atgcgagtaa 3180

taggatacga aggggatgat ttccttagaa cagatttgta ttatgtgaga aataaaaaac 3240

gatatattaa taataaccaa aaagatagta tagagaagct agctgagatt ctttctctat 3300

taggatcagt aaactctttc caacctttag aagcctatag agatgagttc attgataaat 3360

atggtatttt tcaagaggta cctctcctcg aactcataga tgaagaatta ggtattggtt 3420

taccacctga atatcatgaa aacaggcata aaccatcaca accagaaccg agtaatgaat 3480

ttcataggat aaaaaatatt atatcttctt ggcaagcgga ggtgagttta tccaaaaatc 3540

atgagattaa attagatgat gaaaagatta aacatttatt taactcggaa gaattaaata 3600

acattgcggt atcacaagaa ttagaacttt attttaatat tttacttgat gaacacaaag 3660

aaaataaact ctatttaaat cctaactgtg gttctaattc tctttttcaa acatatggaa 3720

gattcaaata ttttttgggt gaaagtgata aagagtacat aaaacaattc tcacatatag 3780

aaaaagatgt tatttttgca gaggttacgt attatcctac ccatggaaaa actgctaaca 3840

ttataaatgc tgaaacatgc tttgattatg agatagcttt tagctgtaat tcgtccaaag 3900

ataataagca ctcaattgcg ttgtctgact tatacgttgg gtcaacatat aactttattt 3960

atcttaagtc aaaaaagttg gataaaatca taataccaaa attaagtcat atgtttaatt 4020

attcaatggg ccctaaagtt tatagatttc ttgttgatat tggtgttcag tatcaaaaac 4080

aatggtctaa tatagatagt ttcttttatg atgctccaat taaaccgagg gttatatata 4140

ataatattgt attatcacct aaaaagtgga acttgaataa aagtattatt ccatttgaat 4200

gtgactctgg agaaaaattc cattactata taaaagattg gaagaagaga tataaggtac 4260

ctgattttat atatttaata gaatttgata ataaacttct gctgaactta gaaaatatct 4320

cacacattaa aattatctat aatgaattta agaagctttc gaataatcaa tcactacatt 4380

ttttagaagc agagaaagag ttgtttaata atcataacaa tgaaatagaa gaatacgttt 4440

ttactttaaa gtcaaagtta catcaattag aacttgattc ttatatgaca aatattcaga 4500

gtgaaatgcc ttttaaatat attggtgaca atgaaaggct taaattctta ggtacagaat 4560

ggatttatgt gaaattatat ggattaggca ctagagaaaa tgaatttata gctgatgaac 4620

ttggctcgtt tataaaaaaa catttagata aaggtactat taagtctagc tttttcatta 4680

gatatagtga ccctaagcag catttacgtg ttagatttaa agttgaaaag aataacattt 4740

tcgatactgt actcccgatt ataataaaat ggttagaaga aatgagaaca aagggttttg 4800

ttaattatta ttgtttcgat ccatacaacc ctgaaataga aagatacgga ggggaacaat 4860

catataagat tgcagaagat ttattttttt gtgactcatt gattgtttct gagtggatac 4920

aaattaacag atctggaaat gttaatattg atgaagtgac ctttggagtt tttaatattc 4980

ttcaactatt aaaattattt aatttaaata ttgaagagca aatgacatgg ttagaaatta 5040

atacaaaacc gcagaaatac gctaataaat tcagaggaaa aaaaagtact ttaattgagc 5100

tggcagactt aatctatact aataagaaac ttgataaata taataaaatc tcaagtatta 5160

tagagatacg agaaaaaact gcaatcacct attctaagag catagaaaat aatccagatt 5220

tcttaataaa aaatattata atgagtgtaa tacacatgca ttgtaacaga ttatatggaa 5280

taaacaggga caaagaaaac ttagttattg ggttagcata tcataccatt aaaaattata 5340

ataatgctct aaagtataat aatgttttaa tccctaacta aattttaatt gaaaggaaag 5400

aattaatgca gaaatgcaag aatcaagatt ttttaaccga tatcctttta gatatagcta 5460

attcaattaa agataagtca gaaaccgaaa aataatactt tggtgatcaa aataatcctg 5520

aagatataat tgaagtagta tcattggcag caggatatcc aggaatttgt gtgtttttag 5580

gttatatgga taaagttttc cctgaagatg gatgggatgt tgtggcacac gactatttaa 5640

cagatttaat ttccttaatt cagactaata aaacatactc actatcccta tttaatgggt 5700

tggcaggtgt tttagtggct cttgaaactt gctctagagg cggaacaaga tatactacaa 5760

taataaatca agtgctaacc ctaattttag aaaactataa acattatttg tctgaggact 5820

tgttgaagct aaagaaagag ggagtgtccg cttctgaata tgatgttata agtggttgga 5880

caggtgttgg aagaacccta ttgcattttg atcaaatatc aaataaagga gatatttcat 5940

taaaaaaagc attgcatgat atagcagaat atttactgtt agttagcagt attgatactc 6000

taagtaatat gccaaattgg cttatatcca atgaaaacct tcctttagat atcgagaaaa 6060

aaacttataa aaaaggatat tttaattctg gcatggcgca tggaatttct ggagttttag 6120

catttttatc gattctgaaa aaagaaaact ataaaataaa gaatttagaa ttaggtatta 6180

aaaacattca aaattggata ttcgaaaagg taaaaacaca caaaggaatt tattatatac 6240

ctaatgtaat tgaaaatgaa gttgttagaa atgaggcttt ttttaaccac agagatgcct 6300

ggtgctatgg tacaccaggt gtgtcaagag caatcttttt atcaggtata gctttgaacg 6360

atcaaagagt taaaatgaaa gccctagaac tgtttaagac atttttttta cgtgatgaga 6420

aagatagaaa aattatatcc cccacaattt gtcatggata tgccggcata ttaatgttga 6480

ctctaagaat gtataaagat actaatgacc gatttctagc tgataaaatg catgatatac 6540

ttaatgaaat gttaactttt tactctcaat taaacttttc caaattcaat aattatgaat 6600

taataagtgg gaaaattgta gaagagaaaa aagtgggttt gttagaagga agtacgggaa 6660

ttgccttagt tttaatttct ttcatttttg gtcaagattc gtgggacaag atatttctga 6720

taagttaaag ggggataaaa ttgatacaag cagtactaga aatcactatt aaatttctaa 6780

cggaattaat ctcttttgaa ttgccagtat atatgtatat ttggttcgca gttcttttca 6840

tattttcaat gatagtcgaa aatgtacaat tggcaatagc taaggatagt atattaaatt 6900

ctattttaaa aagattagga ataaaaattt taagtctatt ggtggtctta attattgggg 6960

ttttaggatc aaactttttt gctcaggttg ttgaagacgc agtcaataaa gaatctaact 7020

tagcttatta tattctgtct actacaatag ctacagcatt gaccataatc gaaccaataa 7080

ttgagacgat gaaaaagttt ataaaagaac aatcaaatct tgcttttgca aattttacag 7140

tcagtgttat ttgtcttact gtaacgtttc aagcagtctc aagcatcttt agtgattatt 7200

ttattttggc caccccttat aaagtggtgt taataatttc atcagcaatt ggtattgtat 7260

atttggtgaa attcgatcaa aataatttag aagttaatag cgaaagtgtt cattcaaata 7320

aaaatgatta atctaagagg ttatacatat gggcaataac catttactat tactgcttaa 7380

acttgttaaa tggccaaaaa aaatattagt atatatttta ttttcattac tatttacaac 7440

tatactatcc ttggtaatac caatattgac taaaaatact gttgatcaat tcactgatgg 7500

aacagtaaat tgggccttaa caataatgtt agcaggttta tttctcataa atattttatt 7560

aagtggatta tctttttatt atctagcatt tttaggtgag aatgcgactt actatatacg 7620

agataaaatg ttttctcata ttttagattt acctctaacg ttttttgata aaaatgaatc 7680

tggtcaaatt gtaagtagag ttatagatga tgttgagtta atgaatactt ttatttctga 7740

caagatacca aactttgctt ctcaattgat agtagttgcc ggatcattaa ttcttctgtt 7800

tgtgctggat tggaaaataa cattacttat gttgatttct gttcctattt cattagctgt 7860

tatgattcca ttggggaata agatgtataa aatttcttta gaaaaacaaa ataatatggc 7920

aatatttact gcaagaataa gtagtgcatt ttcaggaatt agacttacaa aaagttatac 7980

tgcagaaaaa gaagagtatg ggaaaggtaa tgtcatttta aaaagaattt ttaagcttaa 8040

tctaaaagat actaagatac aagcttatat gatgcctatt atgtctactg ttatactaat 8100

gataatatta ttgttagtgg ggtatggggg gataaggtta tcggaaggaa ctatttcacc 8160

aggtaccttt gttgcaataa ttttttactt agttcaattt gttacacctc tttcctctct 8220

gtctaacttt tttactgaat ataaaaaaac actaggtgca acaaaaagat tatatgaaat 8280

atattcacta aaacccgagg ccgaaaataa acaaattagc agtaatgcat ttaacttggg 8340

aaatggggat ctgaaatttg agaacatttc gtttcagtat aacgatcctt cttctgaagt 8400

atttaacaaa ctgaatttgc gttttccaaa aaataagata acagctgttg taggtccttc 8460

tggagctgga aaatcaactt tatttaattt aatagaacgt ttgtatgaag ctcaaaatgg 8520

tgaaatctat tatgaatcaa ctcctataaa agacattgat ttgactgttt ggagaaaaaa 8580

aattggttat gtaatgcaag atactttgat gatcaccggc acgataaaag agaacctgac 8640

gtacggttta tcaggctctg tatctcaaga aactataatt aaatattcta aattagccaa 8700

tgcccatgat tttattatgg agcttccaga taattatgaa acctttattg gagaaagggg 8760

tataaaatta tcaggtggtc aaaaacaaag aattgcaata gcaagagcat taattaaaaa 8820

ccctgaaata cttttattag atgaggcaac ttctaattta gacagtgaaa gtgaaatatt 8880

ggtacaacaa tctataaata gactagttcg agatagaacc actattatta tagctcatag 8940

attgtcaaca attagagatg cagataacat tgtattttta gaaaatggag tggttacagg 9000

aagcggtaat catttggacc taataaataa ccatgaaaaa taccgagagt ttgttaacgg 9060

tcaattgcta aataacactt caataagcgc gagttctcta agttaattgt gcataaataa 9120

tgagaccact tgggtacact ttttatcggt tctataacca taaaaagcag ctgaactttt 9180

agttcagctg ctttttgatt gaatcttgtg actagaaagt caaatgtgaa aataaaactg 9240

ttccaaagtt aatcagaaat ttattttcat attgtatcct ttcagtatca cggtgaaagg 9300

agcatcatat gaaaacatta tggaaagtcc taaaaattgt ttttgtcagc ttggctgctt 9360

tggttttgct tgtatgcgtc tcggtattta tttatcacca tttccagcta aataaggagg 9420

cggcactcct taaagaaaaa ggcacagtag tcgatgttga cggtaaaaag atgaatgtgt 9480

atcaagaggg aagcgggaag gatacgtttg tgtttatgtc tggttcgggt attgctgcgc 9540

ctgcttatga aatgaagggg ctgtacagca agttttcaaa agaaaataag attgctgttg 9600

tagatcgggc aggttatgga tacagtgaag tgtctcacga tgacagagat attgatacgg 9660

tattggaaca gacgaggaaa gcgcttatga aaagcggaaa taagcctcct tatattttaa 9720

tgcctcattc gatatccggg attgaagcga tgtattgggc acagaaatat cctaaggaaa 9780

tcaaggccat tattgcgatg gatattggat tgccccagca gtacgtcacg tataaattga 9840

gcggagttga caggttgaaa gtgagagggt tccacctgtt aacctcgatt ggttttcatc 9900

ggtttatgcc ttccgctgta tataatcctg aggtgatacg gcagtcgttt ttaactgatg 9960

aagaaaaaga aatctataaa gccattaact ttaagcaatt ttttaatgca gatatggagc 10020

atgagctttt acagtcttac caaaacggca gcaaatctgt gaatctgccg gcgccaaagg 10080

aaactcccgt cttgatttta gatgcggtct ctgaccaaaa tagacattca aagtatgcta 10140

tacaaaaccg aaaagattat gaagtgtttg cggctcaatt caatactgcc gatataaagg 10200

aactgagggg aacacacagt atttatttat atcagcctga tcaaatatat aaactgtcca 10260

tggagtttat gagaaaggtt cgctaggatg aagggttatc atattttaat cgttgaagac 10320

gatgtgatga ttggtgattt gctgcaaaag attttgcagc gtgagggata tcgtgtgata 10380

tggaaaacag atggagcgga tgtgctttcg gtgattcaga aggtggattt ggtcattatg 10440

gatgtgatgc tgccgggtga agacgggtat caaatgtctg caaaaatcaa agagctgggg 10500

ctgggcattc cggtaatttt tctttcggcc cgtaatgata tggacagcaa gcttcaaggt 10560

ttacagatcg gcgaggatta tatggtaaag ccctttgatc cgagagagct gctattaaga 10620

atgcggaata tgcttgagca tcattatggc acctttacgc aaatcaaaca tttgtatatt 10680

gatgcggtaa cgaaaaaagt gttcaatgaa agcctgcatg atgaggtatt gtttactgcg 10740

attgagcgga aaattttctt ttatttatat gaaaatagag acagtatcct gacaaaggaa 10800

catttctttg aatatctatg gcagctcgaa gatagaaacc cgaatattgt caatgtgcat 10860

attaaaaaaa tcagagctaa aatcaatgat caagcgggtg agatgattga aaatatatat 10920

ggagaagggt atcggctgaa taccgttgtg aagaaatgaa gctcaagaca aaatatcagt 10980

tgttattgtt tacggctgtc attagtgttc cgatgctgtt gctggcggtc agtgttttga 11040

tgtctgttat ttatgacagc atgtttaaac cgatgaatca tggtatgccc tttcacaggt 11100

<210> 22
<211> 32
<212> PRT
<213> Bacillus subtilis

<220>

<221> MOD_RES
<222> (1)..(1)
<223> Dhb

<220>
<221> MOD_RES
<222> (4)..(4)
<223> Dhb

<220>
<221> MOD_RES
<222> (8)..(8)
<223> Dhb

<220>
<221> MOD_RES
<222> (12)..(12)
<223> Dha

<220>
<221> MOD_RES
<222> (18)..(18)
<223> Dhb

<220>
<221> MOD_RES
<222> (20)..(20)
<223> Dhb

<220>
<221> MOD_RES
<222> (24)..(24)
<223> Dha

<220>
<221> MOD_RES
<222> (27)..(27)
<223> Dha

<220>
<221> MOD_RES
<222> (29)..(29)
<223> Dha

<400> 22
Xaa Trp Ala Xaa Ile Gly Lys Xaa Ile Val Gln Xaa Val Lys Lys Cys
1               5                   10                  15

Arg Xaa Phe Xaa Cys Gly Cys Xaa Leu Gly Xaa Cys Xaa Asn Cys Asn
            20                  25                  30

<210> 23
<211> 171
<212> DNA
<213> Bacillus subtilis

<400> 23
atggagaaga ataatatttt tgacctagac attaataaaa agatggaatc tactagtgag      60

gtttctgctc aaacttgggc tacaatcgga aaaactattg tgcagtctgt gaaaaaatgt      120

agaacattta cttgtggatg ttcgctaggc tcttgcagta actgtaacta a                171

<210> 24
<211> 15
<212> PRT
<213> Bacillus subtilis

<400> 24
Thr Trp Ala Thr Ile Gly Lys Thr Ile Val Gln Ser Val Lys Lys
1               5                   10                  15

<210> 25
<211> 32
<212> PRT
<213> Bacillus subtilis

<400> 25
Thr Trp Ala Thr Ile Gly Lys Thr Ile Val Gln Ser Val Lys Lys Cys
1               5                   10                  15

Arg Thr Phe Thr Cys Gly Cys Ser Leu Gly Ser Cys Ser Asn Cys Asn
            20                  25                  30

<210> 26
<211> 194
<212> PRT
<213> Bacillus subtilis

<400> 26
Leu Ile Gln Ala Val Leu Glu Ile Thr Ile Lys Phe Leu Thr Glu Leu
1               5                   10                  15

Ile Ser Phe Glu Leu Pro Val Tyr Met Tyr Ile Trp Phe Ala Val Leu
            20                  25                  30

Phe Ile Phe Ser Met Ile Val Glu Asn Val Gln Leu Ala Ile Ala Lys
        35                  40                  45

Asp Ser Ile Leu Asn Ser Ile Leu Lys Arg Leu Gly Ile Lys Ile Leu
    50                  55                  60

Ser Leu Leu Val Val Leu Ile Ile Gly Val Leu Gly Ser Asn Phe Phe
65                  70                  75                  80

Ala Gln Val Val Glu Asp Ala Val Asn Lys Glu Ser Asn Leu Ala Tyr
                85                  90                  95

Tyr Ile Leu Ser Thr Thr Ile Ala Thr Ala Leu Thr Ile Ile Glu Pro
            100                 105                 110

Ile Ile Glu Thr Met Lys Lys Phe Ile Lys Glu Gln Ser Asn Leu Ala
        115                 120                 125

Phe Ala Asn Phe Thr Val Ser Val Ile Cys Leu Thr Val Thr Phe Gln
    130                 135                 140

Ala Val Ser Ser Ile Phe Ser Asp Tyr Phe Ile Leu Ala Thr Pro Tyr
145                 150                 155                 160

Lys Val Val Leu Ile Ile Ser Ser Ala Ile Gly Ile Val Tyr Leu Val
                165                 170                 175

Lys Phe Asp Gln Asn Asn Leu Glu Val Asn Ser Glu Ser Val His Ser
            180                 185                 190

Asn Lys Asn Asp
        195

<210> 27
<211> 591
<212> DNA
<213> Bacillus subtilis

<400> 27
ttgatacaag cagtactaga aatcactatt aaatttctaa cggaattaat ctcttttgaa      60

ttgccagtat atatgtatat ttggttcgca gttcttttca tattttcaat gatagtcgaa     120

aatgtacaat tggcaatagc taaggatagt atattaaatt ctattttaaa aagattagga     180

ataaaaattt taagtctatt ggtggtctta attattgggg ttttaggatc aaactttttt     240

gctcaggttg ttgaagacgc agtcaataaa gaatctaact tagcttatta tattctgtct     300

actacaatag ctacagcatt gaccataatc gaaccaataa ttgagacgat gaaaaagttt     360

ataaaagaac aatcaaatct tgcttttgca aattttacag tcagtgttat ttgtcttact     420

gtaacgtttc aagcagtctc aagcatcttt agtgattatt ttattttggc caccccttat     480

aaagtggtgt taataatttc atcagcaatt ggtattgtat atttggtgaa attcgatcaa     540

aataatttag aagttaatag cgaaagtgtt cattcaaata aaaatgatta a              591

<210> 28
<211> 24
<212> PRT
<213> Bacillus subtilis

<400> 28
Met Glu Lys Asn Asn Ile Phe Asp Leu Asp Ile Asn Lys Lys Met Glu
1               5                   10                  15

Ser Thr Ser Glu Val Ser Ala Gln
            20

<210> 29
<211> 56
<212> PRT
<213> Bacillus subtilis

<400> 29
Met Glu Lys Asn Asn Ile Phe Asp Leu Asp Ile Asn Lys Lys Met Glu
1               5                   10                  15

Ser Thr Ser Glu Val Ser Ala Gln Thr Trp Ala Thr Ile Gly Lys Thr
            20                  25                  30

Ile Val Gln Ser Val Lys Lys Cys Arg Thr Phe Thr Cys Gly Cys Ser
        35                  40                  45

Leu Gly Ser Cys Ser Asn Cys Asn
    50                  55

<210> 30
<211> 324
<212> PRT
<213> Bacillus subtilis

<400> 30
Met Asn Met Thr Thr Asn Ala Leu Pro Asn Gln Phe Ser Trp Asn Ser
1               5                   10                  15

Tyr Lys Val Thr Asn Gly Tyr Arg Leu Tyr Asp Leu Gln Val Gly Ser
            20                  25                  30

Pro Leu Ile Lys Ile Ala Val Ile Asp Ser Gly Tyr Asp Pro Asn His
        35                  40                  45

Ser Glu Ile Thr Gly Asn Ile Asp Lys Lys Lys Ser Cys Ser Phe Val
    50                  55                  60

Asp Tyr Glu Asn Phe Asn Glu Asp Leu Tyr Gly His Gly Thr Ala Val
65                  70                  75                  80

Ile Ser Val Ile Val Gly Lys Asn Gln Val Lys Gly Val Ala Pro Gly
                85                  90                  95

Ile Thr Ile Ile Ser Tyr Lys Val Leu Asp Arg Asn Gly Phe Gly Lys
            100                 105                 110

Ile Glu Trp Val Ile His Ala Ile Leu Lys Ala Ile Glu Asp Cys Val
        115                 120                 125

His Ile Ile Asn Leu Ser His Val Phe Thr Met Phe Gly Leu Gly Asn
    130                 135                 140

Lys Gly Ala Phe Asn Lys Leu Gln Gln Ala Ile Asp Lys Ala Arg Lys
145                 150                 155                 160

Lys Gly Ile Ile Ile Val Gly Ala Ala Gly Asn Gln Gly Val Leu Leu
                165                 170                 175

Asp Met Lys Ser Thr Ser Leu Tyr Pro Thr Ile Lys Gly Ile Asn Tyr
            180                 185                 190

Val Thr Ser Thr Asn Lys Asn Asn Tyr Thr Ser Ser Tyr Ala Asn Ile
        195                 200                 205

Gly Ser Ile Ile Arg Phe Ala Ala Pro Ser Gly Glu Leu Asp Glu Thr
    210                 215                 220

Asn Asn Val Thr Asp Leu Ile Ile Val Ala His Pro Thr Asn Phe Pro
225                 230                 235                 240

Lys Thr Lys Phe Glu Lys Ala Ile Gly Leu Pro Asp Phe Tyr Thr Val
                245                 250                 255

Thr Cys Gly Thr Ser Ile Ser Ala Ala Met Phe Ser Ser Leu Ile Ala
            260                 265                 270

Leu Val Leu Thr Glu Tyr Leu Ile Cys Lys Gln Ile His Leu Ser Glu
        275                 280                 285

Lys Glu Ile Leu Asn Ile Ile Gln Lys Asn Ser Ser Arg Val Lys Asp
    290                 295                 300

Gln Asn Ser Ile Phe Glu Ile Asn Ala Tyr Ser Val Ile Lys Phe Ile
305                 310                 315                 320

Lys Gly Leu Leu

<210> 31
<211> 1036
<212> PRT
<213> Bacillus subtilis

<400> 31
   Met Asn Thr Lys Tyr Leu Ser Ser Asp Leu Phe Met Tyr Arg Lys Pro
   1               5                   10                  15
   Ile Leu Asp Leu Ser Phe Tyr Lys Glu Cys Leu Ala Phe Asp Asn Glu
               20                  25                  30
   Phe Asn Glu Glu Glu Tyr Ile Ala Lys Ile Ser Asn Leu Leu Asn Thr
           35                  40                  45
   Asn Pro Thr Ile Ala Glu Ala Ile Tyr Phe Ala Ser Glu Pro Leu Tyr
       50                  55                  60
   Asn Ser Phe Leu Asn Ile Glu Lys Leu Lys Lys Lys Lys Arg Lys Ser
   65                  70                  75                  80
   Val Ile Asp Ser Met Leu Lys Phe Leu Ile Arg Met Ser Thr Arg Pro
                   85                  90                  95
   Thr Pro Phe Gly Leu Phe Ser Gly Ile Gly Val Glu Thr Phe Ser Gly
               100                 105                 110
   Gln Asp Glu Tyr Leu Thr Lys Asp Ser Glu Val Ile Lys Asn Thr Arg
           115                 120                 125
   Leu Asp Met Cys Trp Ile Tyr Phe Leu Ile Arg Lys Leu Glu Ser Asn
       130                 135                 140
   Leu Ser Ile Leu Glu Gln Leu Thr Val Glu Asn Asn Ser Leu Val Tyr
   145                 150                 155                 160
   Asn His Gly Asp Arg Ile Lys Leu Ser Tyr Gln Ser Leu Leu His Ser
                   165                 170                 175
   Asp Asp Asn Asp Gln Ser Leu Glu Met Leu Thr Leu Arg Lys Thr Ala
               180                 185                 190
   Val Met Asp Tyr Val Tyr Ser Gln Ala Glu Phe Gly Ile Asn Tyr Ser
           195                 200                 205
   Thr Leu Arg Asp Ser Ile Asn Lys Thr Tyr Ser Val Thr Asp Asp Ile
       210                 215                 220
   Ser Asn Asp Phe Leu Asn Ser Leu Leu Lys Asn Glu Val Ile Ile Thr
   225                 230                 235                 240
   Asn Leu Arg Thr Ser Leu Val Asn Ser Asp Pro Phe Glu Asn Ile Leu
                   245                 250                 255
   Asn Ile Ile Ser Lys Met His Asn Val Glu Glu Glu Leu Asn Tyr Leu
               260                 265                 270
   Val Arg Leu Lys Lys Ser Ile Glu Glu Tyr Lys Lys Ser Ser Gly Glu
           275                 280                 285
   Leu Gly Ile Ser Ile Leu Lys Lys Ile Lys Lys Glu Met Arg Val Ile
       290                 295                 300
   Gly Tyr Glu Gly Asp Asp Phe Leu Arg Thr Asp Leu Tyr Tyr Val Arg
   305                 310                 315                 320
   Asn Lys Lys Arg Tyr Ile Asn Asn Asn Gln Lys Asp Ser Ile Glu Lys
                   325                 330                 335
   Leu Ala Glu Ile Leu Ser Leu Leu Gly Ser Val Asn Ser Phe Gln Pro
               340                 345                 350
   Leu Glu Ala Tyr Arg Asp Glu Phe Ile Asp Lys Tyr Gly Ile Phe Gln
           355                 360                 365
   Glu Val Pro Leu Leu Glu Leu Ile Asp Glu Glu Leu Gly Ile Gly Leu
       370                 375                 380
   Pro Pro Glu Tyr His Glu Asn Arg His Lys Pro Ser Gln Pro Glu Pro
   385                 390                 395                 400
   Ser Asn Glu Phe His Arg Ile Lys Asn Ile Ile Ser Ser Trp Gln Ala
                   405                 410                 415
   Glu Val Ser Leu Ser Lys Asn His Glu Ile Lys Leu Asp Asp Glu Lys
               420                 425                 430
   Ile Lys His Leu Phe Asn Ser Glu Glu Leu Asn Asn Ile Ala Val Ser
           435                 440                 445
   Gln Glu Leu Glu Leu Tyr Phe Asn Ile Leu Leu Asp Glu His Lys Glu
       450                 455                 460
   Asn Lys Leu Tyr Leu Asn Pro Asn Cys Gly Ser Asn Ser Leu Phe Gln
   465                 470                 475                 480
   Thr Tyr Gly Arg Phe Lys Tyr Phe Leu Gly Glu Ser Asp Lys Glu Tyr
                   485                 490                 495
   Ile Lys Gln Phe Ser His Ile Glu Lys Asp Val Ile Phe Ala Glu Val
               500                 505                 510
   Thr Tyr Tyr Pro Thr His Gly Lys Thr Ala Asn Ile Ile Asn Ala Glu
           515                 520                 525
   Thr Cys Phe Asp Tyr Glu Ile Ala Phe Ser Cys Asn Ser Ser Lys Asp
       530                 535                 540
   Asn Lys His Ser Ile Ala Leu Ser Asp Leu Tyr Val Gly Ser Thr Tyr
   545                 550                 555                 560
   Asn Phe Ile Tyr Leu Lys Ser Lys Lys Leu Asp Lys Ile Ile Ile Pro
                   565                 570                 575
   Lys Leu Ser His Met Phe Asn Tyr Ser Met Gly Pro Lys Val Tyr Arg
               580                 585                 590
   Phe Leu Val Asp Ile Gly Val Gln Tyr Gln Lys Gln Trp Ser Asn Ile
           595                 600                 605
   Asp Ser Phe Phe Tyr Asp Ala Pro Ile Lys Pro Arg Val Ile Tyr Asn
       610                 615                 620
   Asn Ile Val Leu Ser Pro Lys Lys Trp Asn Leu Asn Lys Ser Ile Ile
   625                 630                 635                 640
   Pro Phe Glu Cys Asp Ser Gly Glu Lys Phe His Tyr Tyr Ile Lys Asp
                   645                 650                 655
   Trp Lys Lys Arg Tyr Lys Val Pro Asp Phe Ile Tyr Leu Ile Glu Phe
               660                 665                 670
   Asp Asn Lys Leu Leu Leu Asn Leu Glu Asn Ile Ser His Ile Lys Ile
           675                 680                 685
   Ile Tyr Asn Glu Phe Lys Lys Leu Ser Asn Asn Gln Ser Leu His Phe
       690                 695                 700
   Leu Glu Ala Glu Lys Glu Leu Phe Asn Asn His Asn Asn Glu Ile Glu
   705                 710                 715                 720
   Glu Tyr Val Phe Thr Leu Lys Ser Lys Leu His Gln Leu Glu Leu Asp
                   725                 730                 735
   Ser Tyr Met Thr Asn Ile Gln Ser Glu Met Pro Phe Lys Tyr Ile Gly
               740                 745                 750
   Asp Asn Glu Arg Leu Lys Phe Leu Gly Thr Glu Trp Ile Tyr Val Lys
           755                 760                 765
   Leu Tyr Gly Leu Gly Thr Arg Glu Asn Glu Phe Ile Ala Asp Glu Leu
       770                 775                 780
   Gly Ser Phe Ile Lys Lys His Leu Asp Lys Gly Thr Ile Lys Ser Ser
   785                 790                 795                 800
   Phe Phe Ile Arg Tyr Ser Asp Pro Lys Gln His Leu Arg Val Arg Phe
                   805                 810                 815
   Lys Val Glu Lys Asn Asn Ile Phe Asp Thr Val Leu Pro Ile Ile Ile
               820                 825                 830
   Lys Trp Leu Glu Glu Met Arg Thr Lys Gly Phe Val Asn Tyr Tyr Cys
           835                 840                 845
   Phe Asp Pro Tyr Asn Pro Glu Ile Glu Arg Tyr Gly Gly Glu Gln Ser
       850                 855                 860
   Tyr Lys Ile Ala Glu Asp Leu Phe Phe Cys Asp Ser Leu Ile Val Ser
   865                 870                 875                 880
   Glu Trp Ile Gln Ile Asn Arg Ser Gly Asn Val Asn Ile Asp Glu Val
                   885                 890                 895
   Thr Phe Gly Val Phe Asn Ile Leu Gln Leu Leu Lys Leu Phe Asn Leu
               900                 905                 910
   Asn Ile Glu Glu Gln Met Thr Trp Leu Glu Ile Asn Thr Lys Pro Gln
           915                 920                 925
   Lys Tyr Ala Asn Lys Phe Arg Gly Lys Lys Ser Thr Leu Ile Glu Leu
       930                 935                 940
   Ala Asp Leu Ile Tyr Thr Asn Lys Lys Leu Asp Lys Tyr Asn Lys Ile
   945                 950                 955                 960
   Ser Ser Ile Ile Glu Ile Arg Glu Lys Thr Ala Ile Thr Tyr Ser Lys
                   965                 970                 975
   Ser Ile Glu Asn Asn Pro Asp Phe Leu Ile Lys Asn Ile Ile Met Ser
               980                 985                 990
   Val Ile His Met His Cys Asn Arg  Leu Tyr Gly Ile Asn  Arg Asp Lys
           995                 1000                 1005
   Glu Asn  Leu Val Ile Gly Leu  Ala Tyr His Thr Ile  Lys Asn Tyr
       1010                 1015                 1020
   Asn Asn  Ala Leu Lys Tyr Asn  Asn Val Leu Ile Pro  Asn
       1025                 1030                 1035

<210> 32
<211> 440
<212> PRT
<213> Bacillus subtilis

<400> 32
Met Gln Lys Cys Lys Asn Gln Asp Phe Leu Thr Asp Ile Leu Leu Asp
1               5                   10                  15

Ile Ala Asn Ser Ile Lys Asp Lys Ser Glu Thr Glu Lys Ile Ile Leu
            20                  25                  30

Gly Asp Gln Asn Asn Pro Glu Asp Ile Ile Glu Val Val Ser Leu Ala
        35                  40                  45

Ala Gly Tyr Pro Gly Ile Cys Val Phe Leu Gly Tyr Met Asp Lys Val
    50                  55                  60

Phe Pro Glu Asp Gly Trp Asp Val Val Ala His Asp Tyr Leu Thr Asp
65                  70                  75                  80

Leu Ile Ser Leu Ile Gln Thr Asn Lys Thr Tyr Ser Leu Ser Leu Phe
                85                  90                  95

Asn Gly Leu Ala Gly Val Leu Val Ala Leu Glu Thr Cys Ser Arg Gly
            100                 105                 110

Gly Thr Arg Tyr Thr Thr Ile Ile Asn Gln Val Leu Thr Leu Ile Leu
        115                 120                 125

Glu Asn Tyr Lys His Tyr Leu Ser Glu Asp Leu Leu Lys Leu Lys Lys
    130                 135                 140

Glu Gly Val Ser Ala Ser Glu Tyr Asp Val Ile Ser Gly Trp Thr Gly
145                 150                 155                 160

Val Gly Arg Thr Leu Leu His Phe Asp Gln Ile Ser Asn Lys Gly Asp
                165                 170                 175

Ile Ser Leu Lys Lys Ala Leu His Asp Ile Ala Glu Tyr Leu Leu Leu
            180                 185                 190

Val Ser Ser Ile Asp Thr Leu Ser Asn Met Pro Asn Trp Leu Ile Ser
        195                 200                 205

Asn Glu Asn Leu Pro Leu Asp Ile Glu Lys Lys Thr Tyr Lys Lys Gly
    210                 215                 220

Tyr Phe Asn Ser Gly Met Ala His Gly Ile Ser Gly Val Leu Ala Phe

225                 230                 235                 240

Leu Ser Ile Leu Lys Lys Glu Asn Tyr Lys Ile Lys Asn Leu Glu Leu
                245                 250                 255

Gly Ile Lys Asn Ile Gln Asn Trp Ile Phe Glu Lys Val Lys Thr His
            260                 265                 270

Lys Gly Ile Tyr Tyr Ile Pro Asn Val Ile Glu Asn Glu Val Val Arg
        275                 280                 285

Asn Glu Ala Phe Phe Asn His Arg Asp Ala Trp Cys Tyr Gly Thr Pro
    290                 295                 300

Gly Val Ser Arg Ala Ile Phe Leu Ser Gly Ile Ala Leu Asn Asp Gln
305                 310                 315                 320

Arg Val Lys Met Lys Ala Leu Glu Leu Phe Lys Thr Phe Phe Leu Arg
                325                 330                 335

Asp Glu Lys Asp Arg Lys Ile Ile Ser Pro Thr Ile Cys His Gly Tyr
            340                 345                 350

Ala Gly Ile Leu Met Leu Thr Leu Arg Met Tyr Lys Asp Thr Asn Asp
        355                 360                 365

Arg Phe Leu Ala Asp Lys Met His Asp Ile Leu Asn Glu Met Leu Thr
    370                 375                 380

Phe Tyr Ser Gln Leu Asn Phe Ser Lys Phe Asn Asn Tyr Glu Leu Ile
385                 390                 395                 400

Ser Gly Lys Ile Val Glu Glu Lys Lys Val Gly Leu Leu Glu Gly Ser
                405                 410                 415

Thr Gly Ile Ala Leu Val Leu Ile Ser Phe Ile Phe Gly Gln Asp Ser
            420                 425                 430

Trp Asp Lys Ile Phe Leu Ile Ser
        435                 440

<210> 33
<211> 196
<212> PRT
<213> Bacillus subtilis

<400> 33
Met Ile Gln Ala Val Leu Glu Ile Thr Ile Lys Phe Leu Thr Glu Leu
1               5                   10                  15

Ile Ser Phe Glu Leu Pro Val Tyr Met Tyr Ile Trp Phe Ala Val Leu
            20                  25                  30

Phe Ile Phe Ser Met Ile Val Glu Asn Val Gln Leu Ala Ile Ala Lys
        35                  40                  45

Asp Ser Ile Leu Asn Ser Ile Leu Lys Arg Leu Gly Ile Lys Ile Leu
    50                  55                  60

Ser Leu Leu Val Val Leu Ile Ile Gly Val Leu Gly Ser Asn Phe Phe
65                  70                  75                  80

Ala Gln Val Val Glu Asp Ala Val Asn Lys Glu Ser Asn Leu Ala Tyr
                85                  90                  95

Tyr Ile Leu Ser Thr Thr Ile Ala Thr Ala Leu Thr Ile Ile Glu Pro
            100                 105                 110

Ile Ile Glu Thr Met Lys Lys Phe Ile Lys Glu Gln Ser Asn Leu Ala
        115                 120                 125

Phe Ala Asn Phe Thr Val Ser Val Ile Cys Leu Thr Val Thr Phe Gln
    130                 135                 140

Ala Val Ser Ser Ile Phe Ser Asp Tyr Phe Ile Leu Ala Thr Pro Tyr
145                 150                 155                 160

Lys Val Val Leu Ile Ile Ser Ser Ala Ile Gly Ile Val Tyr Leu Val
                165                 170                 175

Lys Phe Asp Gln Asn Asn Leu Glu Val Asn Ser Glu Ser Val His Ser
            180                 185                 190

Asn Lys Asn Asp
        195

<210> 34
<211> 585
<212> PRT
<213> Bacillus subtilis

<400> 34
Met Gly Asn Asn His Leu Leu Leu Leu Leu Lys Leu Val Lys Trp Pro
1               5                   10                  15

Lys Lys Ile Leu Val Tyr Ile Leu Phe Ser Leu Leu Phe Thr Thr Ile
            20                  25                  30

Leu Ser Leu Val Ile Pro Ile Leu Thr Lys Asn Thr Val Asp Gln Phe
        35                  40                  45

Thr Asp Gly Thr Val Asn Trp Ala Leu Thr Ile Met Leu Ala Gly Leu
    50                  55                  60

Phe Leu Ile Asn Ile Leu Leu Ser Gly Leu Ser Phe Tyr Tyr Leu Ala
65                  70                  75                  80

Phe Leu Gly Glu Asn Ala Thr Tyr Tyr Ile Arg Asp Lys Met Phe Ser
                85                  90                  95

His Ile Leu Asp Leu Pro Leu Thr Phe Phe Asp Lys Asn Glu Ser Gly
            100                 105                 110

Gln Ile Val Ser Arg Val Ile Asp Asp Val Glu Leu Met Asn Thr Phe
        115                 120                 125

Ile Ser Asp Lys Ile Pro Asn Phe Ala Ser Gln Leu Ile Val Val Ala
    130                 135                 140

Gly Ser Leu Ile Leu Leu Phe Val Leu Asp Trp Lys Ile Thr Leu Leu
145                 150                 155                 160

Met Leu Ile Ser Val Pro Ile Ser Leu Ala Val Met Ile Pro Leu Gly
                165                 170                 175

Asn Lys Met Tyr Lys Ile Ser Leu Glu Lys Gln Asn Asn Met Ala Ile
            180                 185                 190

Phe Thr Ala Arg Ile Ser Ser Ala Phe Ser Gly Ile Arg Leu Thr Lys
        195                 200                 205

Ser Tyr Thr Ala Glu Lys Glu Glu Tyr Gly Lys Gly Asn Val Ile Leu
    210                 215                 220

Lys Arg Ile Phe Lys Leu Asn Leu Lys Asp Thr Lys Ile Gln Ala Tyr
225                 230                 235                 240

Met Met Pro Ile Met Ser Thr Val Ile Leu Met Ile Ile Leu Leu Leu
                245                 250                 255

Val Gly Tyr Gly Gly Ile Arg Leu Ser Glu Gly Thr Ile Ser Pro Gly
            260                 265                 270

Thr Phe Val Ala Ile Ile Phe Tyr Leu Val Gln Phe Val Thr Pro Leu
        275                 280                 285

Ser Ser Leu Ser Asn Phe Phe Thr Glu Tyr Lys Lys Thr Leu Gly Ala
    290                 295                 300

Thr Lys Arg Leu Tyr Glu Ile Tyr Ser Leu Lys Pro Glu Ala Glu Asn
305                 310                 315                 320

Lys Gln Ile Ser Ser Asn Ala Phe Asn Leu Gly Asn Gly Asp Leu Lys
                325                 330                 335

Phe Glu Asn Ile Ser Phe Gln Tyr Asn Asp Pro Ser Ser Glu Val Phe
            340                 345                 350

Asn Lys Leu Asn Leu Arg Phe Pro Lys Asn Lys Ile Thr Ala Val Val
        355                 360                 365

Gly Pro Ser Gly Ala Gly Lys Ser Thr Leu Phe Asn Leu Ile Glu Arg
    370                 375                 380

Leu Tyr Glu Ala Gln Asn Gly Glu Ile Tyr Tyr Glu Ser Thr Pro Ile
385                 390                 395                 400

Lys Asp Ile Asp Leu Thr Val Trp Arg Lys Lys Ile Gly Tyr Val Met
                405                 410                 415

Gln Asp Thr Leu Met Ile Thr Gly Thr Ile Lys Glu Asn Leu Thr Tyr
            420                 425                 430

Gly Leu Ser Gly Ser Val Ser Gln Glu Thr Ile Ile Lys Tyr Ser Lys
        435                 440                 445

Leu Ala Asn Ala His Asp Phe Ile Met Glu Leu Pro Asp Asn Tyr Glu
    450                 455                 460

Thr Phe Ile Gly Glu Arg Gly Ile Lys Leu Ser Gly Gly Gln Lys Gln
465                 470                 475                 480

Arg Ile Ala Ile Ala Arg Ala Leu Ile Lys Asn Pro Glu Ile Leu Leu
                485                 490                 495

Leu Asp Glu Ala Thr Ser Asn Leu Asp Ser Glu Ser Glu Ile Leu Val
            500                 505                 510

Gln Gln Ser Ile Asn Arg Leu Val Arg Asp Arg Thr Thr Ile Ile Ile
        515                 520                 525

Ala His Arg Leu Ser Thr Ile Arg Asp Ala Asp Asn Ile Val Phe Leu

530                 535                 540

Glu Asn Gly Val Val Thr Gly Ser Gly Asn His Leu Asp Leu Ile Asn
545                 550                 555                 560

Asn His Glu Lys Tyr Arg Glu Phe Val Asn Gly Gln Leu Leu Asn Asn
                565                 570                 575

Thr Ser Ile Ser Ala Ser Ser Leu Ser
            580                 585

<210> 35
<211> 30
<212> PRT
<213> Paenibacillus polymyxa

<400> 35
Ala Ser Ile Ile Lys Thr Thr Ile Lys Val Ser Lys Ala Val Cys Lys
1               5                   10                  15

Thr Leu Thr Cys Ile Cys Thr Gly Ser Cys Ser Asn Cys Lys
            20                  25                  30

<210> 36
<211> 34
<212> PRT
<213> Lactococcus lactis

<400> 36
Ile Thr Ser Ile Ser Leu Cys Thr Pro Gly Cys Lys Thr Gly Ala Leu
1               5                   10                  15

Met Gly Cys Asn Met Lys Thr Ala Thr Cys Asn Cys Ser Ile His Val
            20                  25                  30

Ser Lys

<210> 37
<211> 32
<212> PRT
<213> Bacillus subtilis

<400> 37
Trp Lys Ser Glu Ser Leu Cys Thr Pro Gly Cys Val Thr Gly Ala Leu
1               5                   10                  15

Gln Thr Cys Phe Leu Gln Thr Leu Thr Cys Asn Cys Lys Ile Ser Lys
            20                  25                  30

<210> 38
<211> 57

<212> PRT
<213> Lactococcus lactis

<400> 38
Met Ser Thr Lys Asp Phe Asn Leu Asp Leu Val Ser Val Ser Lys Lys
1               5                   10                  15

Asp Ser Gly Ala Ser Pro Arg Ile Thr Ser Ile Ser Leu Cys Thr Pro
            20                  25                  30

Gly Cys Lys Thr Gly Ala Leu Met Gly Cys Asn Met Lys Thr Ala Thr
        35                  40                  45

Cys Asn Cys Ser Ile His Val Ser Lys
    50                  55


<210> 39
<211> 56
<212> PRT
<213> Bacillus subtilis

<400> 39
Met Ser Lys Phe Asp Asp Phe Asp Leu Asp Val Val Lys Val Ser Lys
1               5                   10                  15

Gln Asp Ser Lys Ile Thr Pro Gln Trp Lys Ser Glu Ser Leu Cys Thr
            20                  25                  30

Pro Gly Cys Val Thr Gly Ala Leu Gln Thr Cys Phe Leu Gln Thr Leu
        35                  40                  45

Thr Cys Asn Cys Lys Ile Ser Lys
    50                  55


<210> 40
<211> 52
<212> PRT
<213> Staphylococcus epidermidis

<400> 40
Met Glu Ala Val Lys Glu Lys Asn Asp Leu Phe Asn Leu Asp Val Lys
1               5                   10                  15

Val Asn Ala Lys Glu Ser Asn Asp Ser Gly Ala Glu Pro Arg Ile Ala
            20                  25                  30

Ser Lys Phe Ile Cys Thr Pro Gly Cys Ala Lys Thr Gly Ser Phe Asn
        35                  40                  45

Ser Tyr Cys Cys
    50

<210> 41
<211> 55
<212> PRT
<213> Staphylococcus epidermidis

<400> 41
Met Asn Asn Ser Leu Phe Asp Leu Asn Leu Asn Lys Gly Val Glu Thr
1               5                   10                  15

Gln Lys Ser Asp Leu Ser Pro Gln Ser Ala Ser Val Leu Lys Thr Ser
            20                  25                  30

Ile Lys Val Ser Lys Lys Tyr Cys Lys Gly Val Thr Leu Thr Cys Gly
        35                  40                  45

Cys Asn Ile Thr Gly Gly Lys
    50                  55

<210> 42
<211> 56
<212> PRT
<213> Staphylococcus epidermidis

<400> 42
Met Glu Asn Lys Lys Asp Leu Phe Asp Leu Glu Ile Lys Lys Asp Asn
1               5                   10                  15

Met Glu Asn Asn Asn Glu Leu Glu Ala Gln Ser Leu Gly Pro Ala Ile
            20                  25                  30

Lys Ala Thr Arg Gln Val Cys Pro Lys Ala Thr Arg Phe Val Thr Val
        35                  40                  45

Ser Cys Lys Lys Ser Asp Cys Gln
    50                  55

<210> 43
<211> 31
<212> PRT
<213> Bacillus subtilis

<220>
<223> /note="N-term 2-oxobutyrate"

<220>
<221> MOD_RES
<222> (3)..(3)
<223> Dhb

<220>
<221> MOD_RES
<222> (7)..(7)
<223> Dhb

<220>
<221> misc_feature
<222> (11)..(15)
<223> /note="Disulphide bridge between residues"

<220>
<221> MOD_RES
<222> (17)..(17)
<223> Abu

<220>
<221> misc_feature
<222> (17)..(20)
<223> /note="Disulphide bridge between residues"

<220>
<221> MOD_RES
<222> (19)..(19)
<223> Abu

<220>
<221> misc_feature
<222> (19)..(22)
<223> /note="Disulphide bridge between residues"

<220>
<221> misc_feature
<222> (23)..(27)
<223> /note="Disulphide bridge between residues"

<220>
<221> misc_feature
<222> (26)..(30)
<223> /note="Disulphide bridge between residues"

<220>
<221> MOD_RES
<222> (28)..(28)
<223> Dha

<400> 43
Trp Ala Xaa Ile Gly Lys Xaa Ile Val Gln Ala Val Lys Lys Ala Arg
1               5                   10                  15

Xaa Phe Xaa Ala Gly Ala Ala Leu Gly Ala Ala Xaa Asn Ala Asn
            20                  25                  30